# EUROPÄISCHE PATENTSCHRIFT

(11) **EP 3 000 486 B1**
(45) Veröffentlichungstag und Bekanntmachung des Hinweises auf die Patenterteilung: **08.11.2017**
(21) Anmeldenummer: 14185893.6
(22) Anmeldetag: 23.09.2014
(51) Int. Cl.: A61L 15/60, C08J 3/24, C08F 6/00, C08J 9/12, D01F 6/36, C08L 33/08, C08J 5/18, C08L 33/02

(54) **Superabsorberproduktion unter Einsatz bestimmter Fördermaschinen**
Super absorber production using certain conveying machines
Production de super-absorbeur utilisant des machines d'extraction définies

(43) Veröffentlichungstag der Anmeldung: 30.03.2016
(73) Patentinhaber: Evonik Degussa GmbH, 45128 Essen (DE)
(72) Erfinder: Wagner, Dirk, 47918 Tönisvorst (DE); Hoefges, Thomas, 70809 Baton Rouge, LA (US); Klapperich, Udo, 53547 Leubsdorf/Rhein (DE); Van Stiphoudt, Manfred, 47906 Kempten (DE); Rudolph, Henry, 47809 Krefeld (DE); Soppe, Frank, 47608 Geldern (DE); Harren, Jörg, 52499 Baesweiler (DE); Furno, Franck, block 1006 Al-Jasra (BH); Loeker, Frank, 47798 Krefeld (DE)

(56) Entgegenhaltungen:
- EP-A1- 1 462 473
- EP-A1- 2 135 669
- EP-A2- 1 097 946
- EP-A2- 1 118 633
- WO-A1-2007/037454
- WO-A1-2013/072268
- JP-A- 2004 352 849

## Beschreibung

Die vorliegende Erfindung liegt auf dem Gebiet der wasserabsorbierenden Polymerpartikel. Sie betrifft insbesondere ein Verfahren zur Herstellung der wasserabsorbierenden Polymerpartikel unter Einsatz von Fördermaschinen aus der Gruppe der mechanischen Stetigförderer.

Bei der Entwicklung von wasserabsorbierenden oberflächenvernetzten Polymerpartikeln ist es grundsätzlich erstrebenswert, ein möglichst hohes Quellvermögen bei Kontakt mit Flüssigkeit zu erreichen, um eine möglichst große Menge an Flüssigkeit aufnehmen zu können. Weiterhin wird grundsätzlich auch eine hinreichende Gelfestigkeit angestrebt. Dabei kommt es nicht nur darauf an, dass das Polymer Flüssigkeit unter nachfolgender Einwirkung eines Druckes zurückhalten kann, nachdem das Polymer frei quellen konnte. Besonders wichtig ist auch die Fähigkeit der wasserabsorbierenden oberflächenvernetzten Polymerpartikeln, Flüssigkeiten auch bei einem gleichzeitig ausgeübten Druck aufzunehmen, wie es unter praktischen Gesichtspunkten geschieht. Eine optimale Flüssigkeitsabsorption muss auch dann gewährleistet sein, wenn z.B. ein Baby oder eine Person auf einem Sanitärartikel sitzt oder liegt oder wenn es, z. B. durch Beinbewegung, zur Entwicklung von Scherkräften kommt.

Diese spezifische Absorptionseigenschaft wird als Absorption gegen Druck ("Absorbency Against Pressure") oder kurz, AAP bezeichnet und kann gemäß der Edana-Methode ERT 442.2-02 (ERT = Edana Recommended Test; EDANA =European Disposables and Nonwovens Association) bestimmt werden. Der für wasserabsorbierende oberflächenvernetzte Polymerpartikel angegebene AAP-Wert wird dabei maßgeblich von dem aufgewendeten Druck, z. B. 4,83 kPa bestimmt. Bei der Herstellung von wasserabsorbierenden oberflächenvernetzten Polymerpartikeln ist es somit immer ein erstrebenswertes Ziel, einen möglichst guten AAP-Wert zu realisieren.

Die konkrete Aufgabe dieser Erfindung lag deshalb darin, die Bereitstellung von wasserabsorbierenden oberflächenvernetzten Polymerpartikeln mit möglichst gutem AAP-Wert zu ermöglichen, wobei der AAP-Wert als Absorption gegen einen Druck von 4,83 kPa gemäß der von der EDANA (European Disposables and Nonwovens Association) empfohlenen Testmethode Nr. 442.2-02 "Absorption under pressure" bestimmt wird.

Überraschenderweise konnte im Rahmen dieser Erfindung gefunden werden, dass der AAP-Wert durch Förderprozesse während und nach der Produktion der wasserabsorbierenden Polymerpartikel deutlich beeinträchtigt werden kann.

Es konnte überraschenderweise gefunden werden, dass Förderschritte vor dem Oberflächenvernetzungsschritt sowie insbesondere nach der Oberflächenvernetzung, wie z.B. auch der Förderschritt in die Endproduktsilos einen signifikanten Einfluss auf den AAP-Wert der wasserabsorbierenden Polymerpartikel haben können und zwar insofern, dass der AAP-Wert beeinträchtigt werden kann.

Es konnte überraschenderweise gefunden werden, dass der Einsatz bestimmter Fördermaschinen bei diesen Förderschritten die Bereitstellung von wasserabsorbierenden Polymerpartikeln mit möglichst gutem AAP-Wert gewährleisten kann, da auf diese Weise eine Beeinträchtigung des AAP-Wertes verhindert werden kann. Dabei handelt es sich um Fördermaschinen aus der Gruppe der mechanischen Stetigförderer mit Zugmittel, nämlich um Rohrkettenförderer sowie um Becherwerke, wobei erfindungsgemäß ein Rohrkettenförderer eingesetzt wird.

Gegenstand der vorliegenden Erfindung ist somit ein Verfahren zur Herstellung von wasserabsorbierenden oberflächenvernetzten Polymerpartikeln nach Anspruch 1.

Dadurch wird die Bereitstellung von wasserabsorbierenden oberflächenvernetzten Polymerpartikeln mit möglichst gutem AAP-Wert ermöglicht. Beispielsweise kann dagegen der Einsatz von pneumatischen Förderern -wie es beispielsweise EP 2135669 A1 lehrt - überraschenderweise zu einer Beeinträchtigung des AAP führen bei ansonsten gleicher Verfahrensführung.

Neben dem besonders wichtigen Vorteil, dass der AAP nicht beeinträchtigt wird, geht die vorliegende Erfindung noch mit weiteren Vorteilen einher. So wird die Partikelgrößenverteilung durch das erfindungsgemäße Verfahren nicht negativ beeinflusst, wohingegen z.B. insbesondere bei pneumatischer Förderung negative Effekte für die Partikelgrößenverteilung beobachtet werden können. Die Permeabilität (insbesondere SFC) sowie Gelbettpermeabilität (insbesondere GBP) der erfindungsgemäß resultierenden wasserabsorbierenden oberflächenvernetzten Polymerpartikel werden nicht negativ beeinträchtigt. Die Entstehung von Stäuben und deren Austrag in die Umgebung kann minimiert werden. Insgesamt kann der Abluftstrom z.B. im Vergleich zum Einsatz pneumatischer Förderungen signifikant reduziert werden.

Fördermaschinen aus der Gruppe mechanischer Stetigförderer mit Zugmittel sind an sich bekannt. Eine Fördermaschine aus der Gruppe mechanischer Stetigförderer mit Zugmittel, ist das Becherwerk.

Das Becherwerk und seine Funktionsweise sind an sich bekannt und im Rahmen dieser Erfindung kann auf die bekannten, kommerziell verfügbaren Becherwerke zurückgegriffen werden. Ein Becherwerk ist eine Fördermaschine, die insbesondere für die vertikale Förderung von Schüttgut verwendet wird, aber auch für die horizontale Förderung von Schüttgut als auch für die Kombination aus vertikaler und horizontaler Förderung eingesetzt werden kann.

Ein beheizbares Becherwerk zur Förderung von Superabsorberpartikel ist in EP 1118633 A2 offenbart. Das Becherwerk läuft in einem Rohr, welches mit Dampf beaufschlagt wird und somit als Begleitheizung dient. Als Tragmittel für die Superabsorber dienen auschließlich die Becher.

Eine vertikale Förderung im Sinne dieser Erfindung ist eine Förderung, die einen Höhenunterschied überwindet, insbesondere einen Höhenunterschied von mindestens einem Meter, vorzugsweise von mindestens zwei Metern. Eine Obergrenze kann z.B. bei 25 Metern oder z.B. bei 10 Metern oder z.B. bei 5 Metern liegen.

Sogenannte Senkrechtbecherwerke haben starr am Zugmittel befestigte Becher; sie fördern über steil ansteigende (z.B. Steigungswinkel ≥ 70°) oder senkrechte Strecken. Pendelbecherwerke haben wiederum am Zugmittel gelenkig abgehängte Becher, so dass auch horizontale Förderstrecken möglich sind. Pendelbecherwerke ermöglichen somit die Verbindung von waagerechten und senkrechten Förderstrecken. Insbesondere in den Fällen, wo anstelle eines Gurts eine Kette verwendet wird und die Becher beweglich gelagert werden, können also auch schräg geneigte oder horizontale Förderstrecken überbrückt werden.

Bei einem Becherwerk sind in der Regel an einem (insbesondere doppelten oder zentralen) Zugmittel (wie z.B. Kettenstrang, einer Gelenkkette oder auf einem Gurt (Gurtbecherwerk)) Behälter z.B. aus Stahl oder Kunststoff befestigt, die (z.B. über Rutschen oder analoge Vorrichtungen) in der Regel kontinuierlich mit Material beladen werden, dieses in den Behältern (z.B. Trögen oder Bechern) an den Ketten oder dem Gurt (in der Regel aufwärts) fördern und es am Zielpunkt, also vorzugsweise hinter der oberen Umkehrstation abgeben, z.B. auf eine Entlade-Rutsche abkippen.

Ein Senkrechtbecherwerk hat üblicherweise eine obere Station (vorzugsweise mit Antriebsachse, Motor und Getriebe) und eine untere Umkehrstation. Bei einem Senkrechtbecherwerk erfolgt die Aufgabe des Fördergutes in der Regel an der unteren, die Abgabe in der Regel an der oberen Umkehrstelle durch Umkippen.

Bei einem Pendelbecherwerk kann die Gutaufgabe insbesondere an beliebigen Stellen des horizontalen Förderstranges aufgegeben werden. Die Gutabgabe kann an jeder Stelle des horizontal laufenden Förderstranges erfolgen. Zum Entleeren laufen die Becher vorzugsweise gegen einen Anschlag, wodurch sie gekippt werden und sich entleeren. Becherwerke können offen oder geschlossen ausgeführt werden. Insbesondere ist es vorteilhaft, dass Becherwerk im Rahmen dieser Erfindung geschlossen auszuführen und vorzugsweise durch Anschluss an ein Saugsystem in minimalen Unterdruck zu betreiben, was einer bevorzugten Ausführungsform dieser Erfindung betrifft, um eine allfällige Staubbelastung zu minimieren. Vorzugsweise sollte die Fördergeschwindigkeit 1 m/s nicht überschreiten.

Die minimale Bechergeschwindigkeit hängt insbesondere beim Senkrechtbecherwerk mit dem Gewicht des Fördergutes zusammen, denn die Zentrifugalkraft sollte vorzugsweise genügen, um das Material an der oberen Umlenktrommel auszuschleudern. Die optimale Bechergeschwindigkeit kann der Fachmann ohne Weiteres mit wenigen, orientierenden Versuchen in ganz einfacher Weise ermitteln. Insbesondere bei einer Bechergeschwindigkeit von >5m/s kann das transportierte Gut herausgeschleudert werden. Vorzugsweise kann durch besondere Konstruktion des Bechers das auslaufende Produkt über den Rücken des vorauslaufenden Bechers in die Auslaufschurre rutschen, wodurch vorteilhafterweise auch eine Bechergeschwindigkeit von unter 1m/s, ohne Verschmutzung des Becherinnenraumes, erzielt werden kann.

Vorteilhaft ist weiterhin, dass die Einstellung des zu fördernden Schüttgutmassenstroms keiner aufwendigen Regel- und Steuertechnik bedarf, weil das Schüttgut einfach über die Antriebsmotordrehzahl und die damit zusammenhängende Gurtvortriebsgeschwindigkeit (bzw. Kettenvortriebsgeschwindigkeit) oder mittels der über die Beladeschurre zugeführten Schüttgutmenge geregelt werden kann.

Becherwerke sind kommerziell verfügbar. Hersteller von Becherwerken in Deutschland sind z. B. die Unternehmen Zuther in Karwitz, RUD Ketten in Aalen, Aumund in Rheinberg, Emde Industrietechnik mit Standorten in Nassau an der Lahn und in Wurzen (Sachsen), Beumer in Beckum (Westfalen) und DHT in Ennigerloh (Westfalen).
Senkrechtbecherwerke und/oder Pendelbecherwerke sind erfindungsgemäß ganz besonders bevorzugte Fördermaschinen aus der Gruppe mechanischer Stetigförderer mit Zugmittel, die im Rahmen dieser Erfindung einsetzbar sind.
Es ist möglich, ein Senkrechtbecherwerk mit einem Schneckenförderer zu kombinieren, um somit z.B. eine Kombination aus vertikaler und horizontaler Förderung zu realisieren. Schneckenförderer und ihre Funktionsweise sind an sich bekannt und im Rahmen dieser Erfindung kann auf die bekannten, kommerziell verfügbaren Schneckenförderer zurückgegriffen werden. Diese bestehen im Wesentlichen aus einem geschlossenen, ruhenden Rohr oder halbrundförmigen Trog als Tragorgan und einer rotierenden Förderschnecke, die das einzige bewegliche Bauteil darstellt, als Schuborgan. Weitere Baugruppen eines Schneckenförderers sind insbesondere die Gutaufgabe- und Gutabgabestellen, die Antriebseinheit und bei Bedarf Zwischenlager.
Die Förderschnecke ist üblicherweise als Welle mit darauf befestigter durchgehender Schneckenwendel - z.B. entweder aus endlos gewalztem Bandstahl oder aus ausgeschnittenen und gezogenen Blechronden - gestaltet. Als Abwandlung dieser meist genutzten Standardausführung kann die Förderschnecke z.B. auch mit Doppelwendel, konischer Wendel oder Wendel mit veränderlicher Schneckenganghöhe ausgeführt sein. Die Verwendung von Schneckenförderer zur Förderung von Superabsorber ist in EP 2135669 A1 erwähnt.

Eine besondere Fördermaschine aus der Gruppe der Stetigförderer mit Zugmittel, die im Rahmen dieser Erfindung eingesetzt wird, ist der Rohrkettenförderer. Rohrkettenförderer und ihre Funktionsweise sind an sich bekannt und im Rahmen dieser Erfindung kann auf die bekannten, kommerziell verfügbaren Rohrkettenförderer zurückgegriffen werden. Mit dem Rohrkettenförderer ist eine waagerechte, senkrechte oder diagonale (und auch untereinander kombinierbare) Förderung möglich.
Der Rohrkettenförderer setzt sich grundsätzlich aus drei wesentlichen Komponenten zusammen, nämlich einem Rohr als Tragmittel, einer Kette mit daran befestigten Stau- und Mitnehmerscheiben als Zugmittel sowie einer Antriebsstation. Das Grundprinzip des Rohrkettenförderers basiert darauf, dass sich eine Kette mit befestigten Stau- und Mitnehmerscheiben bzw. Transportscheiben in einem Rohr bewegt.

Die Kette bewegt sich als endlos umlaufendes Zugmittel im Rohr. Spannt sich die Kette nicht allein (z.B. in Folge Schwerkraft) muss zusätzlich eine Spannstation eingebaut werden. Bei Umlenkungen der Linienführung (z.B. horizontal auf vertikal) kommen Umlenkstationen zum Einsatz.

Die Förderung mittels Rohrkettenförderer läuft vorzugsweise so ab, dass zu Beginn das zu fördernde Schüttgut an einem Einlauf in die Förderrohrleitung gebracht wird. Im Folgenden wird das Schüttgut durch die an der angetriebenen Kette befestigten Transportscheiben in Förderrichtung mitbewegt und letztlich am Ende der Förderstrecke, vorzugsweise unterhalb der Antriebsstation, am Auslauf wieder abgegeben.

Zum Einstellen des Schüttgutmassenstroms muss lediglich die Kettenantriebsgeschwindigkeit bzw. Schüttgutzufuhr geregelt werden. Die Einstellung eines adäquaten Schüttgutmassenstrom kann mit Hilfe weniger orientierender Versuche vorgenommen werden.

Rohrkettenförderer sind kommerziell verfügbar. Zu nennen sind hier beispielsweise die Schrage Rohrkettensystem GmbH; Deutschland sowie die Horstkötter GmbH & Co. KG, Deutschland.

Es entspricht einer besonders bevorzugten Ausführungsform der Erfindung, wenn bei der Förderung der Polymerpartikel im Rahmen des erfindungsgemäßen Verfahrens sowohl Becherwerk, vorzugsweise Senkrechtbecherwerk und/oder Pendelbecherwerk, sowie Rohrkettenförderer zum Einsatz gelangen.

Es entspricht einer weiteren bevorzugten Ausführumgsform der Erfindung, wenn bei der Förderung der Polymerpartikel im Rahmen des erfindungsgemäßen Verfahrens sowohl Becherwerk, vorzugsweise Senkrechtbecherwerk und/oder Pendelbecherwerk, sowie Rohrkettenförderer als auch Förderschnecke(n) zum Einsatz gelangen.

Bei einer bevorzugten Ausführungsform der Erfindung handelt es sich um ein Verfahren zur Herstellung von wasserabsorbierenden oberflächenvernetzten Polymerpartikeln umfassend in Verfahrensschritt (i) die Polymerisation einer Monomerlösung oder -suspension, enthaltend
(a1) 0,1 bis 99,999 Gew.-%, bevorzugt 10 bis 98,99 Gew.-%, besonders bevorzugt 15 bis 70 Gew.-%, weiter bevorzugt 20 bis 60 Gew.-%, insbesondere 25 bis 50 Gew.-% ethylenisch ungesättigtes, säuregruppentragendes Monomer, das zumindest teilweise neutralisiert sein kann,
(b1) 0,001 bis 10 Gew.-%, bevorzugt 0,01 bis 7 Gew.-%, besonders bevorzugt 0,03 bis 5 Gew.-% , insbesondere 0,05 bis 2 Gew.-%eines oder mehrerer Vernetzer, (c) mindestens einen Initiator,
(d1) 0 bis 70 Gew.-%, bevorzugt 0,01 bis 40 Gew.-%, besonders bevorzugt 0,1 bis 20 Gew.-%, insbesondere 0,5 bis 10 Gew.-% mit den unter (a1) genannten Monomeren copolymerisierbare ethylenisch ungesättigte Monomere,
(e1) 0 bis 30 Gew.-%, bevorzugt 0,1 bis 20 Gew.-% und besonders bevorzugt 0,5 bis 10 Gew.-% wasserlösliche Polymere, sowie
(f) 0 bis 30 Gew.-%, bevorzugt 0,01 bis 7 Gew.-% und besonders bevorzugt 0,05 bis 5 Gew.-% eines oder mehrerer Hilfsstoffe, wobei die Summe der zuvor genannten Gewichtsmengen (a1) bis (f) 100 Gew.-% beträgt,
um ein wasserunlösliches Polymergel zu bilden.

Es entspricht einer bevorzugten Ausführungsform der Erfindung, wenn wenigstens bei einem der Förderschritte (a), (b) vorzugsweise zumindest bei einem Förderschritt (b), insbesondere bei beiden Förderschritten (a), (b) Fördermaschinen aus der Gruppe der mechanischen Stetigförderer mit Zugmittel eingesetzt werden, wobei der Förderschritt (a) vor dem Oberflächennachvernetzungsschritt (vi) liegt und der Förderschritt (b) nach dem Oberflächennachvernetzungsschritt (vi) liegt, wobei die Förderschritte (a) und/oder (b) insbesondere vertikale Förderschritte umfassen. Vertikale Förderschritte dienen zur Überbrückung von Höhenunterschieden, vorzugsweise von mindestens einem Meter, insbesondere von mindestens zwei Metern. Eine Obergrenze kann z.B. bei 25 Metern oder z.B. bei 10 Metern oder z.B. bei 5 Metern liegen.

Insbesondere dann, wenn bei der Förderung, insbesondere umfassend vertikale Förderung, der bereits oberflächennachvernetzten Polymerpartikel auf Fördermaschinen aus der Gruppe der mechanischen Stetigförderer mit Zugmittel zurückgegriffen wird, kann die Bereitstellung von wasserabsorbierenden oberflächenvernetzten Polymerpartikeln mit möglichst gutem AAP-Wert ermöglicht werden.

Es hat sich gezeigt, dass das fertige Endprodukt, also die wasserabsorbierenden oberflächenvernetzten Polymerpartikel, weiterhin einer Beeinträchtigung des AAP-Wertes unterliegen kann und zwar in Abhängigkeit davon, wie die Förderschritte dieses Endproduktes in die Endproduktsilos oder Silofahrzeuge hinein gestaltet werden. Wird dabei auf den erfindungsgemäßen Einsatz von Fördermaschinen aus der Gruppe der mechanischen Stetigförderer mit Zugmittel zurückgegriffen, so kann die Bereitstellung von wasserabsorbierenden oberflächenvernetzten Polymerpartikeln mit möglichst gutem AAP-Wert noch besser ermöglicht werden.

Es entspricht einer weiteren bevorzugten Ausführungsform der Erfindung, wenn bei dem Förderschritt (c), insbesondere umfassend vertikale Förderung, Fördermaschinen aus der Gruppe der mechanischen Stetigförderer mit Zugmittel eingesetzt werden, wobei der Förderschritt (c) den Transport des fertigen Endproduktes, also der wasserabsorbierenden oberflächenvernetzten Polymerpartikeln, betrifft, also den Transport in die Endproduktsilos oder Silofahrzeuge hinein betrifft. Der Förderschritt (c) liegt somit nicht vor dem Schritt (vii), also der optionalen Nachbehandlung und/oder Kühlung des oberflächenvernetzten Polymers.

Weiterhin entspricht es einer bevorzugten Ausführungsform der Erfindung, wenn zumindest der Förderschritt (b), vorzugsweise zumindest die Förderschritte (b) und (c), vorteilhafterweise zumindest die Förderschritte (a), (b) und (c), insbesondere alle Förderschritte, insbesondere umfassend vertikale Förderung, im Rahmen des erfindungsgemäßen Verfahrens unter Einsatz von Fördermaschinen aus der Gruppe der mechanischen Stetigförderer mit Zugmittel erfolgt.

Insbesondere entspricht es einer ganz besonders bevorzugten Ausführungsform der Erfindung, wenn in dem erfindungsgemäßen Verfahren ab Schritt (vi) als Fördermaschinen im Wesentlichen Rohrkettenförderer und/oder Becherwerk, insbesondere für die vertikale Förderung, eingesetzt werden und im Wesentlichen auf pneumatische Fördermaßnahmen verzichtet wird. Vorteilhafterweise kann zumindest in einem der Förderschritte zusätzlich ein Schneckenförderer eingesetzt werden. "Im Wesentlichen Rohrkettenförderer und/oder Becherwerk einzusetzen" bedeutet hierbei, dass zumindest > 50%, vorzugsweise > 60%, vorteilhafterweise > 70%, in weiter vorteilhafter Weise > 80%, in noch weiter vorteilhafter Weise > 85%, in wiederum vorteilhafterer Weise > 90% und insbesondere > 95%, wie z.B. 100%, der zurückzulegenden (insbesondere vertikalen) Transportstrecke mit Becherwerk und/oder Rohrkettenförderer bewältigt wird.

"Im Wesentlichen auf pneumatische Fördermaßnahmen zu verzichten" bedeutet hierbei, dass zumindest > 50%, vorzugsweise > 60%, vorteilhafterweise > 70%, in weiter vorteilhafter Weise > 80%, in noch weiter vorteilhafter Weise > 85%, in wiederum vorteilhafterer Weise > 90% und insbesondere > 95% wie z.B. 100%, der zurückzulegenden (insbesondere vertikalen) Transportstrecke ohne Zuhilfenahme pneumatischer Fördertechnik bewältigt wird.

Es konnte im Rahmen dieser Erfindung gefunden werden, dass sich der erfindungsgemäße Einsatz der Fördermaschinen insbesondere dann hervorragend bewährt und zu besonders guten Ergebnissen führt, wenn bei der Polymerisation der Monomerlösung oder -suspension ein Blähmittel eingesetzt wird. Dadurch kann ein geschäumtes wasserunlösliches Polymergel gebildet werden. Geschäumtes Polymergel ist an sich bekannt. Geschäumtes Polymergel kann z.B. bei der Anwesenheit von Gasbläschen in der Reaktionsmasse bei der Polymerisation resultieren. In der Patentliteratur werden viefältige Methoden beschrieben, um zu geschäumtem Polymergel zu gelangen. Insbesondere umfasst geschäumtes Polymergel gasförmige Bläschen, die von festen oder flüssigen Wänden, insbesondere festen Wänden, eingeschlossen sind.

Das Blähmittel kann bereits vor der Polymerisation in der Monomerlösung oder -suspension enthalten sein und/oder der polymerisierenden Mischung während der Polymerisation zugegeben werden.

Der Einsatz von Blähmittel dient im Sinne dieser Erfindung grundsätzlich dazu, geschäumtes wasserunlösliches Polymergel bereitstellen zu können, um somit vorzugsweise zu Polymerpartikeln mit erhöhter Porosität und vergrößerter Oberfläche zu gelangen. Der Einsatz von Blähmitteln bei der Herstellung wasserabsorbierender oberflächenvernetzter Polymerpartikel ist an sich bekannt.

Als Blähmittel wird im Sinne dieser Erfindung alles das bezeichnet, was zur Erzeugung von Schäumen dienen kann. Zur Erzeugung von Schäumen kann z.B. ein Gas in die flüssige Monomerlösung oder -suspension eingeblasen werden oder man erreicht Schaumbildung durch heftiges Schlagen, Schütteln, Verspritzen oder Rühren der Flüssigkeit, wie hier also der flüssigen Monomerlösung oder -suspension. Weiterhin kann die Schaumbildung auf chemischen Reaktionen, die unter Gasentwicklung ablaufen, beruhen also z.B. aus der Anwesenheit von Verbindungen resultieren, die z.B. unter dem Einfluß von Wärme und/oder in Gegenwart von Wasser, Gase (wie z. B. N2 oder CO2) abspalten.

Es entspricht einer bevorzugten Ausführungsform der Erfindung, wenn als Blähmittel ein Gas, wie vorzugsweise N2 oder CO2, insbesondere CO2, eingesetzt wird, oder eine Verbindung mit Gasfreisetzungsvermögen, wie insbesondere Carbonatsalze.

Die Verbindung mit Gasfreisetzungsvermögen, wie vorzugsweise Carbonatsalze, insbesondere Soda kann in fester Form oder auch in gelöster Form, z.B. in wässriger Lösung zum Einsatz gelangen. Sie kann der Monomerlösung oder -suspension vor oder während der Polymerisation zugegeben werden.

Als Blähmittel können insbesondere alle Carbonate aus der Gruppe von Lithium-, Natrium-, Kalium-, Rubidium-, Cäsiumcarbonat, oder höherwertige Metallionen wie Beryllium-, Calcium-, Magnesium-, Strontiumcarbonat oder Mischungen dieser verwendet werden. Als weitere Verbindungen können auch granulierte Carbonate eingesetzt werden, die auch als Mischsalze aus einem Carbonat und/oder Percarbonat mit einem weiteren als Deckschicht fungierenden Salz wie z. B. einer Sulfatverbindung hergestellt werden können. Erfindungsgemäß können die Blähmittel insbesondere eine Partikelgröße von 10 µm bis 900 µm, bevorzugt 50 µm bis 500 µm und besonders bevorzugt von 100 µm bis 450 µm aufweisen.

Bei der Zugabe von Blähmitteln, wie beispielsweise Soda, entstehen Bläschen bzw. sind Bläschen vorhanden. Erfindungsgemäß ist deshalb bei Einsatz von Blähmitteln auch der Einsatz von Tensiden vorteilhaft, um diese Bläschen zu stabilisieren. Der Einsatz von Tensiden in Kombination mit dem Blähmittel ermöglicht somit den Zugang zu einer besonders vorteilhaften feinporigen Struktur. Der Einsatz von Tensiden kann selbstverständlich auch unabhängig vom Blähmitteleinsatz erfolgen.
Weiterhin entspricht es somit einer bevorzugten Ausführungsform der Erfindung, wenn die Monomerlösung oder -suspension mindestens ein Tensid enthält.

Das Tensid kann insbesondere ein nicht ionisches, ionisches oder amphoteres Tensid sein, wobei auch Tensidmischungen einsetzbar sind.

Tenside sind dem Fachmann an sich bekannt. Bei den erfindungsgemäß einsetzbaren Tensiden handelt es sich insbesondere um solche grenzflächenaktive Verbindungen, die die Oberflächenspannung von Wasser senken können, vorzugsweise unter 70mN/m, besonders bevorzugt unter 68 mN/m, ganz bevorzugt unter 67 mN/m, jeweils gemessen bei 23°C als 0,103gew.-%ige Lösung in Wasser.

Insbesondere handelt es sich um ein Tensid, das zumindest eine polymerisierbare Gruppe aufweist und somit bei der Polymerisation der Monomerlösung -oder suspension mit in das resultierende Polymere einpolymerisiert werden kann. Vorzugsweise handelt es sich um ein ethylenisch ungesättigtes Tensid. Geeignete ethylenisch ungesättigte Gruppen sind beispielsweise Allylether-, Vinylether-, Acrylester- und Methacrylester-Gruppen.

Insbesondere weist das Tensid zumindest eine endständige Kohlenstoff-Kohlenstoff-Doppelbindung auf.

Bevorzugt einsetzbare Tenside weisen zumindest das folgende Strukturelement auf:

CH₂=CH-CH₂-O-(CH₂-CH₂-O)ₙ-

mit n 2 bis 20, vorzugsweise 4 bis 12 und insbesondere 5 bis 8.

Weitere, bevorzugt einsetzbare Tenside weisen zumindest das folgende Strukturelement auf

CH₂=CH-CH₂-O- (EO)ₙ-(PO)ₘ-,

mit EO= Ethylenoxid-Strukturelement und
n= 0 bis 25, vorteilhafterweise 2 bis 20, vorzugsweise 4 bis 12 und insbesondere 5 bis 8, PO= Propylenenoxid-Strukturelement und
m= 0 bis 25, vorteilhafterweise 2 bis 20, vorzugsweise 3 bis 12 und insbesondere 4 bis 7,
wobei die EO- und PO- Bausteine, sofern vorhanden, in gemischter Form, blockweise oder statistisch verteilt sein können.

Weitere, bevorzugt einsetzbare Tenside weisen zumindest das folgende Strukturelement auf

CH2=CR¹-CO-O- (EO)ₙ-(PO)ₘ -,

mit R¹= Wasserstoff, Methyl oder Ethyl, vorzugsweise Methyl oder Ethyl, ganz besonders bevorzugt Methyl,
EO= Ethylenoxid-Strukturelement und
n= 0 bis 25, vorteilhafterweise 2 bis 20, vorzugsweise 4 bis 12 und insbesondere 5 bis 8, PO= Propylenenoxid-Strukturelement und
m= 0 bis 25, vorteilhafterweise 2 bis 20, vorzugsweise 3 bis 12 und insbesondere 4 bis 7,
wobei die EO- und PO- Bausteine, sofern vorhanden, in gemischter Form, blockweise oder statistisch verteilt sein können.

Bevorzugt einsetzbare Tenside genügen z.B. folgender Formel:

CH₂=CH-CH₂-O-(CH₂-CH₂-O)ₙ-Z

mit n 2 bis 20, vorzugsweise 4 bis 12 und insbesondere 5 bis 8,
Z= eine nichtionische Endgruppe wie z.B. Acetyl-, Alkyl- , -OH, ethylenisch ungesättigte Gruppe (beispielsweise Allylether-, Vinylether-, Acrylester- und Methacrylester-Gruppe) oder aber eine ionische Endgruppe, wie beispielsweise quartäre Amin-, Phosphat- oder Sulfat-Gruppe,
oder genügen z.B. folgender Formel

   CH2=CH-CH2-O- (EO)n-(PO)m-Z,

   mit EO= Ethylenoxid-Strukturelement und
   n= 2 bis 20, vorzugsweise 4 bis 12 und insbesondere 5 bis 8,
   PO= Propylenenoxid-Strukturelement und
   m= 0 bis 25, vorteilhafterweise 2 bis 20, vorzugsweise 3 bis 12 und insbesondere 4 bis 7, wobei die EO- und PO- Bausteine, sofern vorhanden, in gemischter Form, blockweise oder statistisch verteilt sein können,
   Z= eine nichtionische Endgruppe wie z.B. Acetyl-, Alkyl- , -OH, ethylenisch ungesättigte Gruppe (beispielsweise Allylether-, Vinylether-, Acrylester- und Methacrylester-Gruppe) oder aber eine ionische Endgruppe, wie beispielsweise quartäre Amin-, Phosphat- oder Sulfat-Gruppe,
   oder genügen z.B. folgender Formel

      CH₂=CR¹-CO-O- (EO)ₙ-(PO)ₘ-Z,

      mit R¹= Wasserstoff, Methyl oder Ethyl, vorzugsweise Methyl oder Ethyl, ganz besonders bevorzugt Methyl,
      EO= Ethylenoxid-Strukturelement und
      n= 2 bis 20, vorzugsweise 4 bis 12 und insbesondere 5 bis 8,
      PO= Propylenenoxid-Strukturelement und
      m= 0 bis 25, vorteilhafterweise 2 bis 20, vorzugsweise 3 bis 12 und insbesondere 4 bis 7, wobei die EO- und PO- Bausteine, sofern vorhanden, in gemischter Form, blockweise oder statistisch verteilt sein können,
      Z= eine nichtionische Endgruppe wie z.B. Acetyl-, Alkyl- , -OH, ethylenisch ungesättigte Gruppe (beispielsweise Allylether-, Vinylether-, Acrylester- und Methacrylester-Gruppe) oder aber eine ionische Endgruppe, wie beispielsweise quartäre Amin-, Phosphat- oder Sulfat-Gruppe.

Bevorzugt einsetzbare Tenside werden auch in der Patentanmeldung WO 2013/072268 A1 beschrieben. Die darin genannten Tenside sind auch im Rahmen dieser Erfindung einsetzbar.

Vorzugsweise ist das Tensid in einer Menge von >0,001 Gew.-%, vorteilhafterweise 0,01 bis 5 Gew.-%, bevorzugt 0,015 bis 2 Gew.-%, weiter bevorzugt 0,02 bis 1 Gew.-%, insbesondere 0,02 bis 0,5 Gew.-% in der Monomerlösung oder -suspension enthalten. Das Tensid ist im Sinne der Systematik dieser Erfindung vorzugsweise unter die sogenannten Hilfsstoffe (f) zu subsummieren.

Erfindungsgemäß wird infolge der Zugabe von Blähmittel vor und/oder während Schritt (i), also der Polymerisation, eine vorzugsweise fein-poröse Struktur erzielt und es können somit insbesondere Polymerpartikel erhalten werden, die eine größere Oberfläche aufweisen. Dadurch kann eine schnellere Absorption der Flüssigkeit, im Vergleich zu herkömmlichen wasserabsorbierenden oberflächenvernetzten Polymerpartikeln gewährleistet werden. Dies wird über den sogenannten FSR-Wert (FSR = Free Swell Rate) abgebildet. Die Bestimmung der Free Swell Rate = FSR erfolgt gemäß dem in der EP0443627 A2 auf der Seite 12, Z. 22 bis 44 beschriebenen Testverfahren.

Erfindungsgemäß bevorzugte wasserabsorbierende Polymerpartikel haben einen FSR im Bereich von vorzugsweise 0,15 bis 0,65, bevorzugt 0,2 bis 0,50 g/gs. Erfindungsgemäß ist es besonders bevorzugt, wenn der FSR-Wert größer als 0,25 g/gs ist.

Durch die erfindungsgemäßen Förderschritte kann das Erreichen bestmöglicher FSR-Werte gewährleistet werden, wohingegen insbesondere durch pneumatische Förderung dagegen eine Reduktion der FSR-Werte wahrscheinlich ist.

Hydrogele, die im gequollenen Zustand eine hohe Gelfestigkeit aufweisen, zeigen gute Transporteigenschaften für Flüssigkeiten. Eine erhöhte Gelfestigkeit wird in aller Regel durch einen höheren Vernetzungsgrad erreicht, wodurch allerdings die Absorptionskapazität des Produktes verringert wird. Eine übliche Methode zur Erhöhung der Gelfestigkeit stellt die Erhöhung des Vernetzungsgrads an der Oberfläche der Superabsorberpartikel gegenüber dem Inneren der Partikel dar. Dazu werden meist in einem Oberflächennachvernetzungsschritt getrocknete Superabsorberpartikel einer zusätzlichen Vernetzung in der Oberflächenschicht ihrer Partikel unterworfen. Dies entspricht der Oberflächenvernetzung. Durch diese Oberflächennachvernetzung bzw. Oberflächenvernetzung steigt die Vernetzungsdichte in der Schale der Superabsorberpartikel, wodurch die Absorption unter Druckbelastung auf ein höheres Niveau gehoben werden kann.

Es konnte im Rahmen dieser Erfindung gefunden werden, dass insbesondere die durch die Oberflächennachvernetzung erzielbare Verbesserung der Absorption unter Druckbelastung wiederum durch den Einsatz pneumatischer Förderung beeinträchtigt werden kann, aber durch den Einsatz der erfindungsgemäßen Förderung, insbesondere umfassend vertikale Förderung, dagegen nicht beeinträchtigt wird.

Es ist deshalb im Rahmen der vorliegenden Erfindung besonders bevorzugt, nach der Oberflächennachvernetzung in Schritt (vi) möglichst weitgehend auf pneumatische Fördermaßnahmen zu verzichten, insbesondere bei vertikaler Förderung, und stattdessen möglichst vollständig auf mechanische Stetigförderer zurückzugreifen, insbesondere bei vertikaler Förderung. Insbesondere ist es bevorzugt, nach der Oberflächennachvernetzung im Wesentlichen auf pneumatische Fördermaßnahmen zu verzichten und stattdessen im Wesentlichen vollständig auf mechanische Stetigförderer mit Zugmittel zurückzugreifen, insbesondere bei vertikaler Förderung. Auf diese Weise lassen sich bei ansonsten unveränderten Verfahren die bestmöglichen AAP-Werte realisieren.

"Im Wesentlichen auf pneumatische Fördermaßnahmen zu verzichten" bedeutet hierbei, dass zumindest > 50%, vorzugsweise > 60%, vorteilhafterweise > 70%, in weiter vorteilhafter Weise > 80%, in noch weiter vorteilhafter Weise > 85%, in wiederum vorteilhafterer Weise > 90% und insbesondere > 95%, z.B. 100%, der zurückzulegenden, insbesondere vertikalen, Transportstrecke ohne Zuhilfenahme pneumatischer Fördertechnik bewältigt wird.

"Im Wesentlichen vollständig auf mechanische Stetigförderer zurückzugreifen" bedeutet hierbei, dass zumindest > 50%, vorzugsweise > 60%, vorteilhafterweise > 70%, in weiter vorteilhafter Weise > 80%, in noch weiter vorteilhafter Weise > 85%, in wiederum vorteilhafterer Weise > 90% und insbesondere > 95%, z.B. 100%, der zurückzulegenden, insbesondere vertikalen, Transportstrecke unter Zuhilfenahme mechanische Stetigförderer mit Zugmittel bewältigt wird.
Im Folgenden werden noch einige bevorzugte Ausgestaltungsmöglichkeiten des erfindungsgemäßen Verfahrens genauer beschrieben.

Die ethylenisch ungesättigten, säuregruppenhaltigen Monomere (a) können teilweise oder vollständig, bevorzugt teilweise neutralisiert sein. Vorzugsweise sind die ethylenisch ungesättigten, säuregruppenhaltigen Monomere zu mindestens 10 Mol-%, besonders bevorzugt zu mindestens 25 bis 50 Mol-% und darüber hinaus bevorzugt zu 50-90 Mol-% neutralisiert. Die Neutralisation der Monomere kann vor, aber auch nach der Polymerisation erfolgen. Hierbei erfolgt die Teilneutralisierung z.B. zu mindestens 10 Mol-%, besonders bevorzugt zu mindestens 25 bis 50 Mol-% und darüber hinaus bevorzugt zu 50-90 Mol-%. Die Neutralisation kann z.B. mit Alkalimetallhydroxiden, Erdalkalimetallhydroxiden, Ammoniak sowie Carbonaten und Bicarbonaten erfolgen. Daneben ist jede weitere Base denkbar, die mit der Säure ein wasserlösliches Salz bildet. Auch eine Mischneutralisation mit verschiedenen Basen ist denkbar. Bevorzugt ist die Neutralisation mit Ammoniak oder mit Alkalimetallhydroxiden, besonders bevorzugt mit Natriumhydroxid oder mit Ammoniak.

Ferner können bei einem Polymer die freien Säuregruppen überwiegen, so dass dieses Polymer einen im sauren Bereich liegenden pH-Wert aufweist. Dieses saure wasserabsorbierende Polymer kann durch ein Polymer mit freien basischen Gruppen, vorzugsweise Amingruppen, das im Vergleich zu dem sauren Polymer basisch ist, mindestens teilweise neutralisiert werden. Diese Polymere werden in der Literatur als *"Mixed-Bed Ion-Exchange Absorbent Polymers"* (MBIEA-Polymere) bezeichnet und sind unter anderem in der WO 99/34843 offenbart. In der Regel stellen MBIEA-Polymere eine Zusammensetzung dar, die zum einen basische Polymere, die in der Lage sind, Anionen auszutauschen, und andererseits ein im Vergleich zu dem basischen Polymer saures Polymer, das in der Lage ist, Kationen auszutauschen, beinhalten. Das basische Polymer weist basische Gruppen auf und wird typischerweise durch die Polymerisation von Monomeren erhalten, die basische Gruppen oder Gruppen tragen, die in basische Gruppen umgewandelt werden können. Bei diesen Monomeren handelt es sich vor allen Dingen um solche, die primäre, sekundäre oder tertiäre Amine oder die entsprechenden Phosphine oder mindestens zwei der vorstehenden funktionellen Gruppen aufweisen. Zu dieser Gruppe von Monomeren gehören insbesondere Ethylenamin, Allylamin, Diallylamin, 4-Aminobuten, Alkyloxycycline, Vinylformamid, 5-Aminopenten, Carbodiimid, Formaldacin, Melamin und dergleichen, sowie deren sekundäre oder tertiäre Aminderivate.

Bevorzugte ethylenisch ungesättigte, säuregruppenhaltige Monomere (a) sind Acrylsäure, Methacrylsäure, Ethacrylsäure, α-Chloracrylsäure, α-Cyanoacrylsäure, β-Methylacrylsäure (Crotonsäure), α-Phenylacrylsäure, β-Acryloxypropionsäure, Sorbinsäure, α-Chlorsorbinsäure, 2'-Methylisocrotonsäure, Zimtsäure, p-Chlorzimtsäure, β-Stearylsäure, Itaconsäure, Citraconsäure, Mesaconsäure, Glutaconsäure, Aconitsäure, Maleinsäure, Fumarsäure, Tricarboxyethylen und Maleinsäureanhydrid, wobei Acrylsäure sowie Methacrylsäure besonders und Acrylsäure darüber hinaus bevorzugt sind.

Neben diesen carboxylatgruppenhaltigen Monomeren sind als ethylenisch ungesättigte, säuregruppenhaltige Monomere (a) des Weiteren ethylenisch ungesättigte Sulfonsäuremonomere oder ethylenisch ungesättigte Phosphonsäuremonomere bevorzugt.

Als ethylenisch ungesättigte Sulfonsäuremonomere sind Allylsulfonsäure oder aliphatische oder aromatische Vinylsulfonsäuren oder acrylische oder methacrylische Sulfonsäuren bevorzugt einsetzbar. Als aliphatische oder aromatische Vinylsulfonsäuren sind Vinylsulfonsäure, 4-Vinylbenzylsulfonsäure, Vinyltoluolsulfonsäure und Stryrolsulfonsäure bevorzugt. Als Acryl- bzw. Methacrylsulfonsäuren sind Sulfoethyl(meth)acrylat, Sulfopropyl(meth)acrylat, 2-Hydroxy-3-methacryloxypropylsulfonsäure sowie (Meth)Acrylamidoalkylsulfonsäuren wie 2-Acrylamido-2-methylpropansulfonsäure bevorzugt.

Als ethylenisch ungesättigte Phosphonsäuremonomere sind Vinylphosphonsäure, Allylphosphonsäure, Vinylbenzylphosponsäure, (Meth)acrylamidoalkylphosphonsäuren, Acrylamidoalkyldiphosphonsäuren, phosponomethylierte Vinylamine und (Meth)acryl-phosphonsäurederivate bevorzugt.

Auch ethylenisch ungesättigte, einen protonierten Stickstoff enthaltende Monomere sind im Rahmen dieser Erfindung einsetzbar. Als ethylenisch ungesättigte, einen protonierten Stickstoff enthaltende Monomere sind vorzugsweise Dialkylaminoalkyl(meth)acrylate in protonierter Form, beispielsweise Dimethylaminoethyl(meth)acrylat-Hydrochlorid oder Dimethylaminoethyl(meth)acrylat-Hydro-sulfat, sowie Dialkylaminoalkyl-(meth)acrylamide in protonierter Form, beispielsweise Dimethylaminoethyl(meth)acrylamid-Hydrochlorid, Diemethylaminopropyl(meth)acrylamid-Hydrochlorid, Diemethylaminopropyl(meth)acrylamid-Hydrosulfat oder Dimethylaminoethyl-(meth)acrylamid-Hydrosulfat bevorzugt.

Auch ethylenisch ungesättigte, einen quarternierten Stickstoff enthaltende Monomere sind im Rahmen dieser Erfindung einsetzbar. Als ethylenisch ungesättigte, einen quarternierten Stickstoff enthaltende Monomere sind Dialkylammoniumalkyl(meth)acrylate in quarternisierter Form, beispielsweise Trimethylam-moniumethyl(meth)acrylat-Methosulfat oder Di-methylethylammoniumethyl(meth)acrylat-Ethosulfat sowie (Meth)acrylamidoalkyldialkylamine in quarternisierter Form, beispielsweise (Meth)acrylamidopropyltrimethylammoniumchlorid, Trimethylammoniumethyl(meth)acrylat-Chlorid oder (Meth)acrylamidopropyltrimethylammoniumsulfat bevorzugt.

Als mit den zuvor genannten säuregruppenhaltigen Monomeren (a) copolymerisierbare ethylenisch ungesättigte Monomere (d) sind insbesondere Acrylamide und Methacrylamide bevorzugt, wobei der Einsatz der Monomere (d) nur optional ist.

Bevorzugte (Meth)acrylamide sind neben Acrylamid und Methacrylamid alkylsubstituierte (Meth)acrylamide oder aminoalkylsubstituierte Derivate des (Meth)acrylamids, wie N-Methylol(meth)acrylamid, N,N-Dimethylamino(meth)- acrylamid, Dimethyl(meth)acrylamid oder Diethyl(meth)acrylamid Mögliche Vinylamide sind beispielsweise N-Vinylamide, N-Vinylformamide, N-Vinylacetamide, N-Vinyl-N-Methylacetamide, N-Vinyl-N-methylformamide, Vinylpyrrolidon. Unter diesen Monomeren besonders bevorzugt ist Acrylamid.

Des Weiteren sind als ethylenisch ungesättigte, mit (a) copolymerisierbaren Monomere (d) in Wasser dispergierbare Monomere bevorzugt. Als in Wasser dispergierbare Monomere sind Acrylsäurester und Methacrylsäurester, wie Methyl(meth)acrylat, Ethyl(meth)acrylat, Propyl(meth)acrylat oder Butyl(meth)acrylat, sowie Vinylacetat, Styrol und Isobutylen bevorzugt.

Erfindungsgemäß bevorzugte Vernetzer (b) sind Verbindungen, die mindestens zwei ethylenisch ungesättigte Gruppen innerhalb eines Moleküls aufweisen (Vernetzerklasse I), Verbindungen, die mindestens zwei funktionelle Gruppen aufweisen, die mit funktionellen Gruppen der Monomeren (a) oder (d) in einer Kondensationsreaktion (=Kondensationsvernetzer), in einer Additionsreaktion oder in einer Ringöffnungsreaktion reagieren können (Vernetzerklasse II), Verbindungen, die mindestens eine ethylenisch ungesättigte Gruppe und mindestens eine funktionelle Gruppe, die mit funktionellen Gruppen der Monomeren (a) oder (d) in einer Kondensationsreaktion, in einer Additionsreaktion oder in einer Ringöffnungsreaktion reagieren kann (Vernetzerklasse III), aufweisen, oder polyvalente Metallkationen (Vernetzerklasse IV). Dabei wird durch die Verbindungen der Vernetzerklasse I eine Vernetzung der Polymere durch die radikalische Polymerisation der ethylenisch ungesättigten Gruppen des Vernetzermoleküls mit den ethylenisch ungesättigten Monomeren (a) oder (d) erreicht, während bei den Verbindungen der Vernetzerklasse II und den polyvalenten Metallkationen der Vernetzerklasse IV eine Vernetzung der Polymere durch Kondensationsreaktion der funktionellen Gruppen (Vernetzerklasse II) bzw. durch elektrostatische Wechselwirkung des polyvalenten Metallkations (Vernetzerklasse IV) mit den funktionellen Gruppen der Monomere (a) oder (d) erreicht wird. Bei den Verbindungen der Vernetzerklasse III erfolgt dementsprechend eine Vernetzung des Polymers sowohl durch radikalische Polymerisation der ethylenisch ungesättigten Gruppe als auch durch Kondensationsreaktion zwischen der funktionellen Gruppe des Vernetzers und den funktionellen Gruppen der Monomeren (a) oder (d).

Bevorzugte Verbindungen der Vernetzerklasse I sind Poly(meth)acrylsäureester, die beispielsweise durch die Umsetzung eines Polyols, wie beispielsweise Ethylenglykol, Propylenglykol, Trimethylolpropan, 1,6-Hexandiol, Glycerin, Pentaerythrit, Polyethylenglykol oder Polypropylenglykol, eines Aminoalkohols, eines Polyalkylenpolyaminens, wie beispielsweise Diethylentriamin oder Triethylentetraamin, oder eines alkoxylierten Polyols mit Acrylsäure oder Methacrylsäure gewonnen werden. Als Verbindungen der Vernetzerklasse I sind des Weiteren Polyvinylverbindungen, Poly(meth)allyverbindungen, (Meth)acrylsäureester einer Monovinylverbindung oder (Meth)acrylsäureester einer Mono(meth)allyl- verbindung, vorzugsweise der Mono(meth)allylverbindungen eines Polyols oder eines Aminoalkohols, bevorzugt. In diesem Zusammenhang wird auf DE 195 43 366 und DE 195 43 368 verwiesen.

Als Verbindungen der Vernetzerklasse I seien als Beispiel genannt Alkenyldi(meth)acrylate, beispielsweise Ethylenglykoldi(meth)acrylat, 1,3-Propylenglykoldi(meth)acrylat, 1,4-Butylenglykoldi(meth)acrylat, 1,3-Butylenglykoldi(meth)acrylat, 1,6-Hexandioldi(meth)acrylat, 1,10-Decandioldi(meth)acrylat, 1,12-Dodecandioldi(meth)acrylat, 1,18-Octadecandioldi(meth)acrylat, Cyclopentandioldi(meth)acrylat, Neopentylglykoldi(meth)acrylat, Methylendi(meth)acrylat oder Pentaerythritdi(meth)acrylat, Alkenyldi(meth)acrylamide, beispielsweise N-Methyldi(meth)acrylamid, N,N'-3-Methylbutylidenbis(meth)acrylamid, N,N'-(1,2-Di-hydroxyethylen)bis(meth)acrylamid, N,N'-Hexamethylenbis(meth)acryl-acrylamid oder N,N'-Methylenbis(meth)acrylamid, Polyalkoxydi(meth)acrylate, beispielsweise Diethylenglykoldi-(meth)acrylat, Triethylenglykoldi(meth)acrylat, Tetraethylenglykoldi(meth)acrylat, Dipropylenglykoldi(meth)acrylat, Tripropylenglykoldi(meth)acrylat oder Tetrapropylenglykoldi-(meth)acrylat, Bisphenol-A-di(meth)acrylat, ethoxyliertes Bisphenol-A-di(meth)acrylat, Benzylidindi(meth)acrylat, 1,3-Di(meth)acryloyloxy-propanol-2, Hydrochinondi(meth)acry- lat, Di(meth)acrylatester des vorzugsweise mit 1 bis 30 Mol Alkylenoxid pro Hydroxylgruppe oxyalkylierten, vorzugsweise ethoxylierten Trimethylolpropans, Thioethylenglykoldi(meth)-acrylat, Thiopropylenglykoldi(meth)acrylat, Thiopolyethylenglykoldi(meth)acrylat, Thiopolypropylenglykoldi(meth)acrylat, Divinylether, beispielsweise 1,4-Butandioldivinylether, Divinylester, beispielsweise Divinyladipat, Alkandiene, beispielsweise Butadien oder 1,6-Hexadien, Divinylbenzol, Di(meth)allylverbindungen, beispielsweise Di(meth)allylphthalat oder Di(meth)allylsuccinat, Homo- und Copolymere von Di(meth)allyldimethylammonium-chlorid und Homo- und Copolymere von Diethyl(meth)allyl- aminomethyl(meth)acrylat-ammoniumchlorid, Vinyl-(meth)acryl-Verbindungen, beispielsweise Vinyl(meth)acrylat, (Meth)allyl-(meth)acryl-Verbindungen, beispielsweise (Meth)allyl(meth)acrylat, mit 1 bis 30 Mol Ethylenoxid pro Hydroxylgruppe ethoxyliertes (Meth)allyl(meth)acrylat, Di(meth)allylester von Polycarbonsäuren, beispielsweise Di(meth)allylmaleat, Di(meth)allyfumarat, Di(meth)allylsuccinat oder Di(meth)allylterephthalat, Verbindungen mit 3 oder mehr ethylenisch ungesättigten, radikalisch polymerisierbaren Gruppen wie beispielsweise Glycerintri(meth)acrylat, (Meth)acrylatester des mit vorzugsweise 1 bis 30 Mol Ethylenoxid pro Hydroxylgruppe oxyethylierten Glycerins, Trimethylolpropantri(meth)acrylat, Tri(meth)acrylatester des vorzugsweise mit 1 bis 30 Mol Alkylenoxid pro Hydroxylgruppe oxyalkylierten, vorzugsweise ethoxylierten Trimethylolpropans, Trimethacrylamid, (Meth)allylidendi(meth)acrylat, 3-Allyloxy-1,2-propandioldi(meth)acrylat, Tri- (meth)allylcyan-urat, Tri(meth)allylisocyanurat, Pentaerythrittetra(meth)acrylat, Pentaerythrittri(meth)acrylat, (Meth)acrylsäureester des mit vorzugsweise 1 bis 30 Mol Ethylenoxid pro Hydroxylgruppe oxyethylierten Pentaerythrits, Tris(2-hydroxyethyl)isocyanurattri(meth)acrylat, Trivinyltrimellitat, Tri(meth)allylamin, Di(meth)allylalkylamine, beispielsweise Di(meth)allylmethylamin, Tri-(meth)allylphosphat, Tetra(meth)allylethylendiamin, Poly(meth)allylester, Tetra(meth)allyloxi-ethan oder Tetra(meth)allylammoniumhalide.

Als Verbindung der Vernetzerklasse II sind Verbindungen bevorzugt, die mindestens zwei funktionelle Gruppen aufweisen, die in einer Kondensationsreaktion (=Kondensaionsvernetzer), in einer Additionsreaktion oder in einer Ringöffnungsreaktion mit den funktionellen Gruppen der Monomere (a) oder (d), bevorzugt mit Säuregruppen, der Monomeren (a), reagieren können. Bei diesen funktionellen Gruppen der Verbindungen der Vernetzerklasse II handelt es sich vorzugsweise um Alkohol-, Amin-, Aldehyd-, Glycidyl-, Isocyanat-, Carbonat- oder Epichlorfunktionen.

Als Verbindung der Vernetzerklasse II seien als Beispiele genannt Polyole, beispielsweise Ethylenglykol, Polethylenglykole wie Diethylenglykol, Triethylenglykol und Tetraethylenglykol, Propylenglykol, Polypropylenglykole wie Dipropylenglykol, Tripropylenglykol oder Tetrapropylenglykol, 1,3-Butandiol, 1,4-Butandiol, 1,5-Pentandiol, 2,4-Pentandiol, 1,6-Hexandiol, 2,5-Hexandiol, Glycerin, Polyglycerin, Trimethylolpropan, Polyoxypropylen, Oxyethylen-Oxypropylen-Blockcopolymere, Sorbitanfettsäureester, Polyoxyethylensorbitanfettsäureester, Pentaerythrit, Polyvinylalkohol und Sorbitol, Aminoalkohole, beispielsweise Ethanolamin, Diethanolamin, Triethanolamin oder Propanolamin, Polyaminverbindungen, beispielsweise Ethylendiamin, Diethylentriaamin, Triethylentetraamin, Tetraethylenpentaamin oder Pentaethylenhexaamin, Polyglycidylether-Verbindungen wie Ethylenglykol-iglycidylether, Polyethylenglykoldiglycidylether, Glycerindiglycidylether, Glycerinpolyglycidy-ether, Pentareritritpolyglycidylether, Propylenglykoldiglycidylether Polypropylenglykoldiglycidylether, Neopentylglykoldiglycidylether, Hexandiolglycidylether, Trimethylolpropanpolyglycidylether, Sorbitolpolyglycidylether, Phtahlsäurediglycidylester, Adipinsäurediglycidylether, 1,4-Phenylen-bis(2-oxazolin), Glycidol, Polyisocyanate, vorzugsweise Diisocyanate wie 2,4-Toluoldiisocyanat und Hexamethylendiisocyanat, Polyaziridin-Verbindungen wie 2,2-Bishydroxymethylbutanol-tris[3-(1-aziridinyl)propionat], 1,6-Hexamethylendiethylenharnstoff und Diphenylmethan-bis-4,4'-N,N'-diethylenharnstoff, Halogenperoxide beispielsweise Epichlor- und Epibromhydrin und α-Methylepichlorhydrin, Alkylencarbonate wie 1,3-Dioxolan-2-on (Ethylencarbonat), 4-Methyl-1,3-dioxolan-2-on (Propylencarbonat), 4,5-Dimethyl-1,3-dioxolan-2-on, 4,4-Dimethyl-1,3-dioxolan-2-on, 4-Ethyl-1,3-dioxolan-2-on, 4-Hydroxymethyl-1,3-dioxolan-2-on, 1,3-Dioxan-2-on, 4-Methyl-1,3-dioxan-2-on, 4,6-Dimethyl-1,3-dioxan-2-on, 1,3-Dioxolan-2-on, Poly-1,3-dioxolan-2-on, polyquartäre Amine wie Kondensationsprodukte von Dimethylaminen und Epichlorhydrin. Als Verbindungen der Vernetzerklasse II sind des Weiteren Polyoxazoline wie 1,2-Ethylenbisoxazolin, Vernetzer mit Silangruppen wie γ-Glycidoxypropyltrimethoxysilan und γ-Aminopropyltrimethoxysilan, Oxazolidinone wie 2-Oxazolidinon, Bis- und Poly-2-oxazolidinone und Diglykolsilikate bevorzugt.

Als Verbindungen der Klasse III sind hydroxyl- oder aminogruppenhaltige Ester der (Meth)acrylsäure, wie beispielsweise 2-Hydroxyethyl(meth)acrylat und 2-Hydroxypropyl(meth)acrylat sowie hydroxyl- oder aminogruppenhaltige (Meth)acrylamide oder Mono(meth)allylverbindungen von Diolen bevorzugt.

Die polyvalenten Metallkationen der Vernetzerklasse IV leiten sich vorzugsweise von ein- oder mehrwertigen Kationen ab, die einwertigen insbesondere von Alkalimetallen, wie Kalium, Natrium, Lithium, wobei Lithium bevorzugt wird. Bevorzugte zweiwertige Kationen leiten sich von Zink, Beryllium, Erdalkalimetallen, wie Magnesium, Calcium, Strontium ab, wobei Magnesium bevorzugt wird. Weiter erfindungsgemäß einsetzbare höherwertige Kationen sind Kationen von Aluminium, Eisen, Chrom, Mangan, Titan, Zirkonium und andere Übergangsmetalle sowie Doppelsalze solcher Kationen oder Mischungen der genannten Salze. Bevorzugt werden Aluminiumsalze und Alaune und deren unterschiedliche Hydrate wie z. B. AlCl₃ × 6H₂O, NaAl(SO₄)₂ × 12 H₂O, KAl(SO₄)₂ × 12 H₂O oder Al₂(SO₄)₃×14-18 H₂O eingesetzt. Besonders bevorzugt werden Al₂(SO₄)₃ und seine Hydrate als Vernetzer der Vernetzungsklasse IV verwendet.

Die im erfindungsgemäßen Verfahren erhältlichen Superabsorberteilchen (das sind die als Verfahrensendprodukt des erfindungsgemäßen Verfahrens resultierenden wasserabsorbierenden Polymerpartikel) sind vorzugsweise durch Vernetzer der folgenden Vernetzerklassen bzw. durch Vernetzer der folgenden Kombinationen von Vernetzerklassen vernetzt: I, II, III, IV; I II; I III; I IV; I II III; I II IV; I III IV; II III IV; II IV oder III IV. Die vorstehenden Kombinationen von Vernetzerklassen stellen jeweils eine bevorzugte Ausführungsform im Sinne der Erfindung dar.

Der Einsatz von Vernetzern der Vernetzerklasse I ist besonders bevorzugt. Unter diesen sind wasserlösliche Vernetzer bevorzugt. In diesem Zusammenhang sind N,N'-Methylen-bisacrylamid, Polyethylenglykoldi(meth)acrylate, Triallylmethylammoniumchlorid, Tetraallylammoniumchlorid sowie mit 9 Mol Ethylenoxid pro Mol Acrylsäure hergestelltes Allylnonaethylenglykolacrylat besonders bevorzugt.

Als optionale wasserlösliche Polymere (e) können wasserlösliche Polymerisate, wie teil- oder vollverseifter Polyvinylalkohol, Polyvinylpyrrolidon, Stärke oder Stärkederivate, Polyglykole oder Polyacrylsäure eingesetzt werden. Das Molekulargewicht dieser Polymere ist unkritisch, solange sie wasserlöslich sind. Bevorzugte wasserlösliche Polymere sind Stärke oder Stärkederivate oder Polyvinylalkohol. Die wasserlöslichen Polymere, vorzugsweise synthetische wie Polyvinylalkohol, können auch als Pfropfgrundlage für die zu polymerisierenden Monomeren dienen.

Als Hilfsstoffe (f) können z.B. organische oder anorganische Partikel wie beispielsweise Geruchsbinder, insbesondere Zeolithe oder Cyclodextrine, Hautpflegesubstanzen, oberflächenaktive Mittel oder Antioxidantien eingesetzt werden. Hilfsstoffe im Sinne dieser Erfindung können auch Tenside sein, die insbesondere zusammen mit Blähmitteln einsetzbar sind.

Zu den bevorzugten organischen Hilfsstoffen gehören Cyclodextrine oder deren Derivate sowie Polysaccharide. Außerdem sind Cellulose und Cellulosederivate wie CMC, Celluloseether bevorzugt. Als Cyclodextrine oder Cyclodextrinderivate sind dabei diejenigen Verbindungen bevorzugt, die in DE-A-198 25 486 auf der Seite 3, Zeile 51 bis Seite 4, Zeile 61 offenbart sind. Besonders bevorzugte Cyclodextrine sind nicht derivatisierte α-, β-, γ- oder δ-Cyclodextrine.

Als anorganische partikuläre Hilfsstoffe können alle Materialen eingesetzt werden, welche üblicherweise zur Modifizierung der Eigenschaften wasserabsorbierender Polymere eingesetzt werden. Zu den bevorzugten anorganischen Hilfsstoffen gehören Sulfate wie Na₂SO₄, Lactate wie etwa Natriumlactat, Silikate, insbesondere Gerüstsilikate wie Zeolithe oder Silikate, die durch Trocknung wässriger Kieselsäurelösungen oder Kieselsolen erhalten wurden, beispielsweise die kommerziell erhältlichen Produkte wie Fällungskieselsäuren und pyrogene Kieselsäuren, beispielsweise Aerosile mit einer Teilchengröße im Bereich von 5 bis 50 nm, vorzugsweise im Bereich von 8 bis 20 nm wie "Aerosil 200" der Evonik Industries AG, Aluminate, Titandioxide, Zinkoxide, Tonmaterialien und weitere dem Fachmann geläufige Mineralien sowie kohlenstoffhaltige anorganische Materialien.

Bevorzugte Silikate sind alle natürlichen oder synthetischen Silikate, die in Hollemann und Wiberg, Lehrbuch der Anorganischen Chemie, Walter de Gruyter-Verlag, 91.-100. Auflage, 1985 auf den Seiten 750 bis 783, als Silikate offenbart sind.

Besonders bevorzugte Silikate sind die Zeolithe. Als Zeolithe können alle dem Fachmann bekannten synthetischen oder natürlichen Zeolithe eingesetzt werden. Bevorzugte natürliche Zeolithe sind Zeolithe aus der Natrolith-Gruppe, der Harmoton-Gruppe, der Mordenit-Gruppe, der Chabasit-Gruppe, der Faujasit-Gruppe (Sodalith-Gruppe) oder der Analcit-Gruppe. Beispiele für natürliche Zeolithe sind Analcim, Leucit, Pollucite, Wairakite, Bellbergite, Bikitaite, Boggsite, Brewsterite, Chabasit, Willhendersonite, Cowlesite, Dachiardite, Edingtonit, Epistilbit, Erionit, Faujasit, Ferrierite, Amicite, Garronite, Gismondine, Gobbinsite, Gmelinit, Gonnardite, Goosecreekit, Harmoton, Phillipsit, Wellsite, Clinoptilolit, Heulandit, Laumontit, Levyne, Mazzite, Merlinoite, Montesommaite, Mordenit, Mesolit, Natrolit, Scolecit, Offretite, Paranatrolite, Paulingite, Perlialite, Barrerite, Stilbit, Stellerit, Thomsonit, Tschernichite oder Yugawaralite. Bevorzugte synthetische Zeolithe sind Zeolith A, Zeolith X, Zeolith Y, Zeolith P oder das Produkt ABSCENTS®.

Als Zeolithe können Zeolithe des so genannten "mittleren" Typs eingesetzt werden, bei denen das SiO₂/AlO₂-Verhältnis kleiner als 10 ist, besonders bevorzugt liegt das SiO₂/AlO₂-Verhältnis dieser Zeolithe in einem Bereich von 2 bis 10. Neben diesen "mittleren" Zeolithen können weiterhin Zeolithe des "hohen" Typs eingesetzt werden, zu denen beispielsweise die bekannten "Molekularsieb"-Zeolithe des Typs ZSM sowie β-Zeolith gehören. Diese "hohen" Zeolithe sind vorzugsweise durch ein SiO₂/AlO₂-Verhältnis von mindestens 35, besonders bevorzugt von einem SiO₂/AlO₂-Verhältnis in einem Bereich von 200 bis 500 gekennzeichnet.

Als Aluminate werden vorzugsweise die in der Natur vorkommenden Spinelle, insbesondere gewöhnlicher Spinell, Zinkspinell, Eisenspinell oder Chromspinell eingesetzt.

Bevorzugtes Titandioxid ist das reine Titandioxid in den Kristallformen Rutil, Anatas und Brookit, sowie eisenhaltige Titandioxide wie beispielsweise Ilmenit, calciumhaltige Titandioxide wie Titanit oder Perowskit.

Bevorzugte Tonmaterialien sind diejenigen, die in Hollemann und Wiberg, Lehrbuch der Anorganischen Chemie, Walter de Gruyter-Verlag, 91.-100. Auflage, 1985 auf den Seiten 783 bis 785, als Tonmaterialien offenbart sind. Besonders bevorzugte Tonmaterialien sind Kaolinit, Illit, Halloysit, Montmorillonit sowie Talk.

Weiterhin sind als anorganische Feinteilchen die Metallsalze der Mono-, Oligo- und Polyphosphorsäuren erfindungsgemäß bevorzugt. Hierunter sind insbesondere die Hydrate bevorzugt, wobei die Mono- bis Deca-Hydrate und Tri-Hydrate besonders bevorzugt sind. Als Metalle kommen insbesondere Alkali- und Erdalkalimetalle in Betracht, wobei die Erdalkalimetalle bevorzugt sind. Hierunter sind Mg und Ca bevorzugt und Mg besonders bevorzugt. Im Zusammenhang mit Phosphaten, Phosphorsäuren und deren Metallverbindungen wird auf Hollemann und Wiberg, Lehrbuch der Anorganischen Chemie, Walter de Gruyter-Verlag, 91.-100. Auflage, 1985, auf den Seiten 651 bis 669 verwiesen.

Bevorzugte kohlenstoffhaltige, jedoch nicht organische Hilfsstoffe sind diejenigen reinen Kohlenstoffe, die in Hollemann und Wiberg, Lehrbuch der Anorganischen Chemie, Walter de Gruyter-Verlag, 91.-100. Auflage, 1985 auf den Seiten 705 bis 708 als Graphite genannt sind. Besonders bevorzugte Graphite sind künstliche Graphite wie beispielsweise Koks, Pyrographit, Aktivkohle oder Ruß.

Der Monomerlösung oder -Suspension bzw. deren Rohstoffen können optional alle bekannten Chelatbildner als Hilfsstoffe zur besseren Kontrolle der Polymerisationsreaktion hinzugefügt werden. Geeignete Chelatbildner sind beispielsweise Phosphorsäure, Diphosphorsäure, Triphosphorsäure, Polyphosphorsäure, Zitronensäure, Weinsäure, sowie deren Salze.

Weiterhin geeignet als Hilfsstoffe sind beispielsweise Iminodiessigsäure, Hydroxyethyliminodiessig-säure, Nitrilotriessigsäure, Nitrilotripropionsäure, Ethylendiamintetraessigsäure, Diethylentriaminpen- taessigsäure, Triethylentetraaminhexaessigsäure, N,N-Bis(2-Hydroxyethyl)glycin und trans-1 ,2- diaminocyclohexantetraessigsäure, sowie deren Salze. Die Einsatzmenge beträgt üblicherweise 1 bis 30.000 ppm bezogen auf die gesamte Monomermenge, vorzugsweise 10 bis 1.000 ppm, bevorzugt 20 bis 600 ppm, mehr bevorzugt 50 bis 400 ppm, am meisten bevorzugt 100 bis 300 ppm.

Die im erfindungsgemäßen Verfahren erhaltenen wasserabsorbierende Polymere sind vorzugsweise dadurch erhältlich, dass zunächst aus den vorgenannten Monomeren und Vernetzern ein Polymergel, auch Hydrogel-Polymer genannt, in partikulärer Form hergestellt wird. Die Herstellung dieses Ausgangsmaterials für die wasserabsorbierenden Polymere kann beispielsweise durch Massepolymerisation, die vorzugsweise in Knetreaktoren wie Extrudern erfolgt, Lösungspolymerisation, Spraypolymerisation, inverse Emulsionspolymerisation oder inverse Suspensionspolymerisation erfolgen. Bevorzugt kann die Lösungspolymerisation in Wasser als Lösemittel durchgeführt werden. Die Lösungspolymerisation kann kontinuierlich oder diskontinuierlich erfolgen. Aus dem Stand der Technik ist ein breites Spektrum von Variationsmöglichkeiten hinsichtlich Reaktionsverhältnisse wie Temperaturen, Art und Menge der Initiatoren als auch der Reaktionslösung bekannt. Typische Verfahren sind in den folgenden Patentschriften beschrieben: US 4,286,082, DE 27 06 135, US 4,076,663, DE 35 03 458, DE 40 20 780, DE 42 44 548, DE 43 23 001, DE 43 33 056, DE 44 18 818.

Als Initiatoren (c) zur Initiierung der Polymerisation können alle unter den Polymerisationsbedingungen Radikale bildende Initiatoren verwendet werden, die üblicherweise bei der Herstellung von Superabsorbern eingesetzt werden. Hierzu gehören thermische Initiatoren, Redoxinitiatoren und Photoinitiatoren, deren Aktivierung durch energiereiche Strahlung erfolgt. Die Polymerisationsinitiatoren können dabei in einer Lösung erfindungsgemäßer Monomere gelöst oder dispergiert enthalten sein. Bevorzugt ist der Einsatz wasserlöslicher Initiatoren.

Als thermische Initiatoren kommen sämtliche dem Fachmann bekannte, unter Temperatureinwirkung in Radikale zerfallende Verbindungen in Betracht. Besonders bevorzugt sind dabei thermische Polymerisationsinitiatoren mit einer Halbwertszeit von weniger als 10 Sekunden, darüber hinaus bevorzugt von weniger als 5 Sekunden bei weniger als 180 ºC, darüber hinaus bevorzugt bei weniger als 140ºC. Dabei sind Peroxide, Hydroperoxide, Wasserstoffperoxid, Persulfate sowie Azoverbindungen besonders bevorzugte thermische Polymerisationsinitiatoren. In manchen Fällen ist es vorteilhaft, Mischungen verschiedener thermischer Polymerisationsinitiatoren zu verwenden. Unter diesen Mischungen sind die aus Wasserstoffperoxid und Natrium- oder Kaliumperoxodisulfat bevorzugt, die in jedem denkbaren Mengenverhältnis eingesetzt werden können. Geeignete organische Peroxide sind vorzugsweise Acetylacetonperoxid, Methylethylketonperoxid, Benzoylperoxid, Lauroylperoxid, Acetylperoxid, Capyrlperoxid, Isopropylperoxydicarbonat, 2-Ethylhexylperoxydicarbonat, t-Butylhydroperoxid, Cumolhydroperoxid, t-Amylperpivalat, t-Butylperpivalat, t-Butylperneohexonat, t-Butylisobutyrat, t-Butylper-2-ethylhexenoat, t-Butylperisononanoat, t-Butylpermaleat, t-Butylperbenzoat, t-Butyl-3,5,5-tri-methylhexanoat und Amylperneodekanoat. Weiterhin sind als thermische Polymerisationsinitiatoren bevorzugt: Azo-Verbindungen, wie Azobisisobutyronitrol, Azobisdimethylvaleronitril, 2,2'-Azobis-(2-amidinopropan)dihydrochlorid, Azo-bis-amidinopropan-dihydrochlord, 2,2'-Azobis-(N,N-dimethylen)isobutyramidin-dihydrochlorid, 2- (Carbamoylazo)- isobutyronitril und 4,4'-Azobis-(4-cyanovaleriansäure). Die genannten Verbindungen werden in üblichen Mengen eingesetzt, vorzugsweise in einem Bereich von 0,01 bis 5, bevorzugt von 0,1 bis 2 Mol-%, jeweils bezogen auf die Menge der zu polymerisierenden Monomere.

Die Redoxinitiatoren enthalten als oxidische Komponente mindestens eine der oben angegebenen Perverbindungen und als reduzierende Komponente vorzugsweise Ascorbinsäure, Glukose, Sorbose, Manose, Ammonium- oder Alkalimetall-hydrogensulfit, - sulfat, -thiosulfat, -hyposulfit oder -sulfid, Metallsalze, wie Eisen-II-ionen oder Silberionen oder Natriumhydroxymethylsulfoxylat. Vorzugsweise wird als reduzierende Komponente des Redoxinitiators Ascorbinsäure oder Natriumpyrosulfit verwendet. Bezogen auf die bei der Polymerisation eingesetzte Menge an Monomeren wird z.B. 1×10⁻⁵ bis 1 Mol-% der reduzierenden Komponente des Redoxinitiators und z.B. 1×10⁻⁵ bis 5 Mol-% der oxidierenden Komponente des Redoxinitiators eingesetzt. Anstelle der oxidierenden Komponente des Redoxinitiators, oder in Ergänzung zu diesem, können ein oder mehrere, vorzugsweise wasserlösliche, Azoverbindungen verwendet werden.

Wenn man die Polymerisation durch Einwirkung energiereicher Strahlung auslöst, verwendet man üblicherweise als Initiator sogenannte Photoinitiatoren. Hierbei kann es sich beispielsweise um sogenannte α-Spalter, H-abstrahierende Systeme oder auch um Azide handeln. Beispiele für solche Initiatoren sind Benzophenon-Derivate wie Michlers-Keton, Phenanthren-Derivate, Fluoren-Derivate, Anthrachinon-Derivate, Thioxanton-Derivate, Cumarin-Derivate, Benzoinether und deren Derivate, Azoverbindungen wie die oben genannten Radikalbildner, substituierte Hexaarylbisimidazole oder Acylphosphinoxide. Beispiele für Azide sind: 2-(N,N-Dimethylamino)-ethyl-4-azidocinnamat, 2-(N,N-Dimethylamino)-ethyl-4-azidonaphthylketon, 2-(N,N-Dimethylamino)-ethyl-4-azidobenzoat, 5-Azido-1-naphthyl-2'-(N,N-dimethylamino)ethylsulfon, N-(4-Sulfonylazidophenyl)malein- imid, N-Acetyl-4-sulfonylazidoanilin, 4-Sulfonylazidoanilin, 4-Azidoanilin, 4-Azidophenacylbromid, p-Azidobenzoesäure, 2,6-Bis(p-azidobenzyliden)cyclo-hexanon und 2,6-Bis-(p-azidobenzyliden)-4-methylcyclohexanon. Die Photoinitiatoren werden, falls sie eingesetzt werden, üblicherweise in Mengen von 0,01 bis 5 Gew.-%, bezogen auf die zu polymerisierenden Monomeren angewendet.

Bevorzugt wird erfindungsgemäß ein Initiatorsystem bestehend aus Wasserstoffperoxid, Natriumperoxodisulfat und Ascorbinsäure eingesetzt. In der Regel wird die Polymerisation mit den Initiatoren in einem Temperaturbereich von 0°C bis 90°C initiiert.

Die Polymerisationsreaktion kann durch einen Initiator oder durch mehrere, zusammenwirkende Initiatoren ausgelöst werden. Weiterhin kann die Polymerisation derart durchgeführt werden, dass man zunächst ein oder mehrere Redoxinitiatoren zusetzt. Im weiteren Polymerisationsverlauf werden dann zusätzlich thermische Initiatoren oder Photoinitiatoren appliziert, wobei im Falle von Photoninitiatoren die Polymerisationsreaktion dann durch die Einwirkung energiereicher Strahlung initiiert wird. Auch die umgekehrte Reihenfolge, also die anfängliche Initiierung der Reaktion mittels energiereicher Strahlung und Photoinitiatoren oder thermischen Initiatoren und eine im weiteren Polymerisationsverlauf erfolgende Initiierung der Polymerisation mittels eines oder mehrerer Redoxinitiatoren ist denkbar.

Um das bei der Polymerisation resultierende Polymergel, das auch Hydrogel-Polymer bezeichnet wird, in eine partikuläre Form zu überführen, kann dieses nach Abtrennung aus der Reaktionsmischung ggf. zunächst zerkleinert werden, z.B. in einem Extruder oder Kneter oder durch sogenannte Wolfen in Zerkleinerungsgeräten nach Bauart eines Fleischwolfes, entspricht Schritt (ii), dann ggf. aufgelockert werden, entspricht Schritt (iia) und dann getrocknet werden, entspricht Schritt (iii).

Die optionale Zerkleinerung gemäß Schritt (ii) kann insbesondere mit einem Zerkleinerungsgerät durchgeführt werden, das vorzugsweise eine Schneideinheit, eine Reißeinheit und/oder eine Wölfeinheit umfasst. Mit Hilfe der Schneideinheit wird das Polymergel geschnitten. Mit Hilfe der Reißeinheit wird das Polymergel zerrissen. Mit Hilfe der Wölfeinheit wird das Polymergel gequetscht. Durch Kombination dieser drei Zerkleinerungsarten kann eine besonders vorteilhafte Zerkleinerung bewirkt werden.

Ein Zerkleinerungsschritt (ii) ist insbesondere dann vorteilhaft, wenn die Monomerlösung oder -Suspension mit Hilfe eines Bandreaktors polymerisiert wird. Im Knetreaktor kann das bei der Polymerisation einer wässrigen Monomerlösung oder -Suspension entstehende Polymergel beispielsweise durch gegenläufige Rührwellen bereits im Kneter selbst kontinuierlich zerkleinert werden.

Bei der optionalen Auflockerung gemäß Schritt (iia) wird das Polymergel mit einer geeigneten Auflockerungseinrichtung aufgelockert. Vorteilhafter Weise ist die Auflockerungseinheit eine rotierende Trommel, vorzugsweise ein Trommelrotationsmischer. Durch die Auflockerung kann die Schüttgutdichte des Polymergels reduziert werden.

Gemäß einer bevorzugten Ausführungsform werden sowohl der Zerkleinerungsschritt (ii) als auch der Auflockerungsschritt (iia) vollzogen.

Bei der Trocknung des Polymergels gemäß Schritt (iii), welches zuvor vorzugsweise in den optionalen Schritten (ii) und (iia) zerkleinert und aufgelockert worden ist, wird der Wassergehalt des Polymergels reduziert. Es kann z.B. bei einer Temperatur in einem Bereich von 20 bis 300ºC, bevorzugt in einem Bereich von 50 bis 250ºC und besonders bevorzugt in einem Bereich von 100 bis 200ºC bis hin zu einem Wassergehalt von z.B. weniger als 40 Gew.-%, bevorzugt von weniger als 20 Gew.-% und darüber hinaus bevorzugt von weniger als 10 Gew.-%, z.B. 2 bis 8 Gew.-%, jeweils bezogen auf das Gesamtgewicht des Polymergel, getrocknet werden, wobei der Restfeuchtegehalt gemäß der von der EDANA empfohlenen Testmethode ERT 430.2-02 bestimmt wird. Bei. Die Trocknung erfolgt vorzugsweise in dem Fachmann bekannten Öfen oder Trocknern, beispielsweise in Bandtrocknern, Hordentrocknern, Drehrohröfen, Wirbelbetttrocknern, Tellertrocknern, Paddeltrocknern oder Infrarottrocknern. Die EDANA-Testmethoden sind beispielsweise erhältlich bei der EDANA, Avenue Eugene Plasky 157, B-1030 Brüssel, Belgien.

Bei einer zu hohen Restfeuchte weist das getrocknete Polymergel eine zu niedrige Glasübergangstemperatur Tg auf und ist nur schwierig weiter zu verarbeiten. Bei einer zu niedrigen Restfeuchte ist das getrocknete Polymergel zu spröde und in den anschließenden Zerkleinerungsschritten fallen unerwünscht große Mengen an Polymerpartikeln mit zu niedriger Partikelgröße ("fines") an. Der Feststoffgehalt des Gels beträgt vor der Trocknung vorzugsweise von 25 und 90 Gew.-%, besonders bevorzugt von 35 bis 70 Gew.-%, ganz besonders bevorzugt von 40 bis 60 Gew.-%.

Das getrocknete Polymergel wird hiernach gemahlen, entspricht Schritt (iv), und klassiert, entspricht Schritt (v), wobei zur Mahlung die üblichen Vorrichtungen, wie üblicherweise ein- oder mehrstufige Walzenstühle, bevorzugt zwei- oder dreistufige Walzenstühle, Stiftmühlen, Hammermühlen oder Schwingmühlen, eingesetzt werden können.

Das Klassieren kann wie bei der Superabsorberherstellung üblich in bekannter Weise ausgeführt werden, wobei insbesondere das Siebklassieren bevorzugt ist. Siebklassierung umfasst die Trennung des teilchenförmigen Gutes nach deren geometrischen Abmessungen mit Hilfe einer Trennfläche (Siebboden), die definierte Öffnungen aufweist. Die Trennflächen können verschieden ausgeführt sein: z.B. Spaltroste, gelochte Platten, Drahtgewebe. Der Siebprozess wird insbesondere unter Relativbewegung zwischen Siebgut und Siebboden ausgeführt.

Die mittlere Partikelgröße der als Produktfraktion abgetrennten Polymerpartikel beträgt vorzugsweise mindestens 150 µm, besonders bevorzugt von 150 bis 800 µm, ganz besonders von 200 bis 750 µm. Die mittlere Partikelgröße der Produktfraktion kann mittels der von der EDANA empfohlenen Testmethode ERT 420.2-02 "Partikel Size Distribution" ermittelt werden, wobei die Massenanteile der Siebfraktionen kumuliert aufgetragen werden und die mittlere Partikelgröße graphisch bestimmt wird. Die mittlere Partikelgröße ist hierbei der Wert der Maschenweite, der sich für kumulierte 50 Gew.-% ergibt. Vorzugsweise werden getrocknete wasserabsorbierende Polymerpartikel mit einem Wassergehalt von weniger als 10 Gew.-%, vorzugsweise von weniger als 5 Gew.%, besonders bevorzugt von weniger als 3 Gew.%, verwendet. Der Wassergehalt kann beispielsweise nach der von der EDANA (European Disposables and Nonwovens Association) empfohlenen Testmethode Nr. 430.2-02 "Moisture content" bestimmt werden.
Der Anteil an Partikeln mit einer Partikelgröße von mindestens 150 µm beträgt vorzugsweise mindestens 90 Gew.-%, besonders bevorzugt mindestens 95 Gew.-%, ganz besonders bevorzugt mindestens 98 Gew.-%.
Polymerpartikel mit zu niedriger Partikelgröße senken die Permeabilität (SFC). Daher sollte der Anteil zu kleiner Polymerpartikel ("fines") niedrig sein. Zu kleine Polymerpartikel werden daher üblicherweise abgetrennt und in das Verfahren rückgeführt. Dies geschieht vorzugsweise vor, während oder unmittelbar nach der Polymerisation, d.h. vor der Trocknung des Polymergels. Die zu kleinen Polymerpartikel können vor oder während der Rückführung mit Wasser und/oder wässrigem Tensid angefeuchtet werden.
Es ist auch möglich auch noch in späteren Verfahrensschritten zu kleine Polymerpartikel abzutrennen, beispielsweise nach der Oberflächennachvernetzung oder einem anderen Beschichtungsschritt.
Der Anteil an Partikeln mit einer Partikelgröße von höchstens 850 µm, beträgt vorzugsweise mindestens 90 Gew.-%, besonders bevorzugt mindestens 95 Gew.-%, ganz besonders bevorzugt mindestens 98 Gew.-%. Der Anteil an Partikeln mit einer Partikelgröße von höchstens 750 µm, beträgt vorzugsweise mindestens 90 Gew.-%, besonders bevorzugt mindestens 95 Gew.-%, ganz besonders bevorzugt mindestens 98 Gew.-%. Polymerpartikel mit zu großer Partikelgröße senken die Anquellgeschwindigkeit. Daher sollte der Anteil zu großer Polymerpartikel ebenfalls niedrig sein.
Zu große Polymerpartikel werden daher üblicherweise abgetrennt und in die Mahlung des getrockneten Polymergels rückgeführt.
Die Polymerpartikel können zur weiteren Verbesserung der Eigenschaften oberflächennachvernetzt werden, insbesondere unter Wärmezufuhr, entspricht Schritt (vii).
In einer bevorzugten Ausführungsform der erfindungsgemäßen Verfahren werden als wasserabsorbierende Polymere Partikel erhalten, die einen Innenbereich und einen den Innenbereich begrenzenden Oberflächenbereich aufweisen. Wobei der Oberflächenbereich eine andere chemische Zusammensetzung als der Innenbereich aufweist oder sich in einer physikalischen Eigenschaft vom Innenbereich unterscheidet. Physikalische Eigenschaften, in denen sich der Innenbereich vom Oberflächenbereich unterscheidet, sind beispielsweise die Ladungsdichte oder der Vernetzungsgrad.
Diese einen Innenbereich und einen den Innenbereich begrenzenden Oberflächenbereich aufweisenden wasserabsorbierenden Polymere sind vorzugsweise dadurch erhältlich, dass oberflächennahe, reaktive Gruppen der Partikel des Polymers nachvernetzt werden. Diese Oberflächvernetzung bzw. Oberflächennachvernetzung kann thermisch, photochemisch oder chemisch erfolgen, insbesondere thermisch.

Als Nachvernetzer bevorzugt sind die im Zusammenhang mit den o.g. Vernetzern (b) genannten Verbindungen der Vernetzerklasse II und IV.

Unter diesen Verbindungen sind als Nachvernetzer besonders bevorzugt Diethylenglykol, Triethylenglykol, Polyethylenglykol, Glyzerin, Polyglyzerin, Propylenglykol, Diethanolamin, Triethanolamin, Polyoxypropylen, Oxyethylen-Oxypropylen-Blockcopolymere, Sorbitanfettsäureester, Polyoxyethylensorbitanfettsäureester, Trimethylolpropan, Pentaerytrit, Polyvinylalkohol, Sorbitol, 1,3-Dioxolan-2-on (Ethylencarbonat), 4-Methyl-1,3-dioxolan-2-on (Propylencarbonat), 4,5-Dimethyl-1,3-dioxolan-2-on, 4,4-Dimethyl-1,3-dioxolan-2-on, 4-Ethyl-1,3-dioxolan-2-on, 4-Hydroxymethyl-1,3-dioxolan-2-on, 1,3-Dioxan-2-on, 4-Methyl-1,3-dioxan-2-on, 4,6-Dimethyl-1,3-dioxan-2-on, 1,3-Dioxolan-2-on, Poly-1,3-dioxolan-2-on.

Besonders bevorzugt wird Ethylencarbonat als Nachvernetzer eingesetzt.

Bevorzugte Ausführungsformen der wasserabsorbierenden Polymere sind diejenigen, die durch Vernetzer der folgenden Vernetzerklassen bzw. durch Vernetzer der folgenden Kombinationen von Vernetzerklassen nachvernetzt sind: II; IV; II IV.

Vorzugsweise wird der Nachvernetzer in einer Menge > 0,001 Gew.-%, vorteilhafterweise in einem Bereich von 0,01 bis 30 Gew.-%, besonders bevorzugt in einer Menge in einem Bereich von 0,1 bis 20 Gew.-%, weiter bevorzugt in einer Menge in einem Bereich von 0,2 bis 5 Gew.-%, insbesondere 0,3 bis 2 Gew.-% jeweils bezogen auf das Gewicht der superabsorbierenden Polymere bei der Nachvernetzung eingesetzt.

Es ist ebenfalls bevorzugt, dass die Nachvernetzung dadurch erfolgt, dass ein Lösemittel, umfassend vorzugsweise Wasser, mit Wasser mischbare organische Lösemittel wie etwa Methanol oder Ethanol oder Mischungen aus mindestens zwei davon, sowie der Nachvernetzer mit dem Aussenbereich der Polymerteilchen bei einer Temperatur in einem Bereich von 30 bis 300°C, besonders bevorzugt in einem Bereich von 100 bis 200°C in Kontakt gebracht werden. Das in Kontakt bringen erfolgt dabei vorzugsweise durch Aufsprühen der Mischung, umfassend Nachvernetzer und Lösemittel auf die Hydrogel-Polymerteilchen und anschließendes Mischen der mit der Mischung in Kontakt gebrachten Hydrogel-Polymerteilchen. Dabei ist der Nachvernetzer in der Mischung vorzugsweise in einer Menge > 0,001 Gew.-%, vorteilhafterweise in einem Bereich von 0,01 bis 70 Gew.-%, besonders bevorzugt in einer Menge in einem Bereich von 0,1 bis 60 Gew.-%, beispielsweise in Mengen von 30 bis 50 Gew.-%, bezogen auf das Gesamtgewicht der Mischung, enthalten.

Als Kondensationsreaktionen kommen vorzugsweise die Bildung von Ester-, Amid-, Imid- oder Urethanbindungen in Betracht, wobei die Bildung von Esterbindung bevorzugt ist.

Insbesondere ist es bevorzugt, wenn vor, während oder nach der Oberflächennachvernetzung zusätzlich zu anderen Oberflächennachvernetzern polyvalente Kationen, entsprechend Vernetzerklasse IV, auf die Partikeloberfläche aufgebracht werden, wobei die Einsatzmenge an polyvalentem Kation beispielsweise 0,001 bis 3 Gew.-%, vorzugsweise 0,005 bis 2 Gew.-%, besonders bevorzugt 0,02 bis 1 Gew.-% jeweils bezogen auf die Polymerpartikel betragen kann.

Die Oberflächennachvernetzung wird in einer bevorzugten Ausführungsform der Erfindung insbesondere so durchgeführt, dass eine Lösung des Oberflächennachvernetzers auf die getrockneten Polymerpartikel aufgesprüht wird. Im Anschluss an das Aufsprühen werden die mit Oberflächennachvernetzer beschichteten Polymerpartikel vorzugsweise thermisch getrocknet, wobei die Oberflächennachvernetzungsreaktion sowohl vor als auch während der Trocknung stattfinden kann.

Das Aufsprühen einer Lösung des Oberflächennachvernetzers kann vorzugsweise in Mischern mit bewegten Mischwerkzeugen, wie Schneckenmischer, Scheibenmischer und Schaufelmischer, durchgeführt werden. Besonders bevorzugt sind Horizontalmischer, wie Schaufelmi- scher, ganz besonders bevorzugt sind Vertikalmischer. Die Unterscheidung in Horizontalmischer und Vertikalmischer erfolgt über die Lagerung der Mischwelle, d.h. Horizontalmischer haben eine horizontal gelagerte Mischwelle und Vertikalmischer haben eine vertikal gelagerte Mischwelle. Geeignete Mischer sind beispielsweise Horizontale Pflugschar® Mischer (Gebr. Lödige Maschinenbau GmbH; Paderborn; Deutschland), Vrieco-Nauta Continuous Mixer (Ho sokawa Micron BV; Doetinchem; Niederlande), Processall Mixmill Mixer (Processall Incorpora- ted; Cincinnati; U.S.A.) und Schugi Flexomix® (Hosokawa Micron BV; Doetinchem; Niederlande). Es ist aber auch möglich, die Oberflächennachvernetzerlösung in einem Wirbelbett aufzusprühen. Die Oberflächennachvernetzer können insbesondere in wässriger Lösung eingesetzt werden. Über den Gehalt an nichtwässrigem Lösungsmittel bzw. Gesamtlösungsmittelmenge kann die Eindringtiefe des Oberflächennachvernetzers in die Polymerpartikel eingestellt werden.

Vorzugsweise können Lösungsmittelgemische eingesetzt werden, beispielsweise Isopropanol/Wasser, 1 ,3-Propandiol/Wasser und Propylenglykol/Wasser, wobei das Mischungsmassenverhältnis vorzugsweise von 20:80 bis 40:60 beträgt.

Die thermische Trocknung kann vorzugsweise in Kontakttrocknern, besonders bevorzugt Schaufeltrocknern, ganz besonders bevorzugt Scheibentrocknern, durchgeführt werden. Geeignete Trockner sind beispielsweise Hosokawa Bepex® Horizontal Paddle Dryer (Hosokawa Micron GmbH; Leingarten; Deutschland), Hosokawa Bepex® Disc Dryer (Hosokawa Micron GmbH; Leingarten; Deutschland), Holo-Flite® dryers (Metso Minerals Industries Inc.; Danville; U.S.A.) und Nara Paddle Dryer (NARA Machinery Europe; Frechen; Deutschland). Überdies können auch Wirbelschichttrockner eingesetzt werden.

Die Trocknung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Besonders vorteilhaft wird in einem Wirbelschichttrockner gemischt und getrocknet. Bevorzugte Trocknungstemperaturen liegen im Bereich 100 bis 250°C, bevorzugt 120 bis 220°C, besonders bevorzugt 130 bis 210°C, ganz besonders bevorzugt 150 bis 200°C. Die bevorzugte Verweilzeit bei dieser Temperatur im Reaktionsmischer oder Trockner beträgt vorzugsweise mindestens 10 Minuten, besonders bevorzugt mindestens 20 Minuten, ganz besonders bevorzugt mindestens 30 Minuten, und üblicherweise höchstens 60 Minuten.

Die vorgenannte thermische Trocknung kann im Rahmen des Schrittes (vi), also der Oberflächennachvernetzung erfolgen.

In einer bevorzugten Ausführungsform der vorliegenden Erfindung können die wasserabsorbierenden Polymerpartikel nach der thermischen Trocknung gekühlt und optional nachbehandelt werden, entspricht Schritt (vii).

Die Kühlung wird vorzugsweise in Kontaktkühlern, besonders bevorzugt Schaufelkühlern, ganz besonders bevorzugt Scheibenkühlern, durchgeführt. Geeignete Kühler sind beispielsweise Hosokawa Bepex® Horizontal Paddle Cooler (Hosokawa Micron GmbH; Leingarten; Deutschland), Hosokawa Bepex® Disc Cooler (Hosokawa Micron GmbH; Leingarten; Deutschland), Holo-Flite® coolers (Metso Minerals Industries Inc.; Danville; U.S.A.) und Nara Paddle Cooler (NARA Machinery Europe; Frechen; Deutschland). Überdies können auch Wirbelschichtkühler eingesetzt werden.

Im Kühler können die wasserabsorbierenden Polymerpartikel z.B. auf 20 bis 150°C, vorzugsweise 40 bis 120°C, besonders bevorzugt 60 bis 100°C, ganz besonders bevorzugt 70 bis 90°C, abgekühlt werden. Während oder nach Kühlung können die Polymerpartikel gewünschtenfalls nachbehandelt werden.

Anschließend können die oberflächennachvernetzten Polymerpartikel erneut klassiert werden, wobei zu kleine und/oder zu große Polymerpartikel abgetrennt und in das Verfahren rückgeführt werden.

Die oberflächennachvernetzten Polymerpartikel können insbesondere zur weiteren Verbesserung der Eigenschaften optional nachbehandelt, nämlich z.B. beschichtet oder nachbefeuchtet werden. Dies kann z.B. während oder nach der Kühlung erfolgen.

Die optionale Nachbefeuchtung wird vorzugsweise bei 30 bis 90°C, besonders bevorzugt bei 35 bis 70°C, ganz besonders bevorzugt bei 40 bis 60°C, durchgeführt. Bei zu niedrigen Temperaturen neigen die wasserabsorbierenden Polymerpartikel zum Verklumpen und bei höheren Temperaturen verdampft bereits merklich Wasser. Die zur Nachbefeuchtung eingesetzte Wassermenge beträgt vorzugsweise von 0,1 bis 10 Gew.-%, besonders bevorzugt von 0,2 bis 8 Gew.-%, ganz besonders bevorzugt von 0,3 bis 5 Gew.-%, jeweils bezogen auf die wasserabsorbierenden Polymerpartikel. Durch die Nachbefeuchtung wird die mechanische Stabilität der Polymerpartikel erhöht und deren Neigung zur statischen Aufladung vermindert. Vorteilhaft wird die Nachbefeuchtung im Kühler nach der thermischen Trocknung durchgeführt. Geeignete Beschichtungen zur Verbesserung der Anquellgeschwindigkeit sowie der Permeabilität (SFC) sind beispielsweise anorganische inerte Substanzen, wie wasserunlösliche Metallsalze, organische Polymere, kationische Polymere sowie zwei- oder mehrwertige Metallkationen. Geeignete Beschichtungen zur Staubbindung sind beispielsweise Polyole sowie Polyethylenglykole. Geeignete Beschichtungen gegen die unerwünschte Verbackungsneigung der Polymerpartikel sind beispielsweise pyrogene Kieselsäure, wie Aerosil® 200, und Tenside, wie Span® 20.

Darüber hinaus können noch weitere Additive und Effektstoffe zugesetzt werden.

Als Additive sind z.B. Trennmittel bevorzugt, wie etwa anorganische oder organische pulverförmige Trennmittel. Diese Trennmittel können vorzugsweise in Mengen in einem Bereich von 0 bis 2 Gew.-%, besonders bevorzugt in einem Bereich von 0,1 bis 1,5 Gew.-%, bezogen auf das Gewicht des wasserabsorbierenden Polymers, eingesetzt werden. Bevorzugte Trennmittel sind Holzmehl, Pulp Fasern, pulverförmige Rinde, Cellulosepulver, mineralische Füllstoffe wie Perlit, synthetische Füllstoffe wie Nylonpulver, Rayonpulver, Diatomerde, Bentonit, Kaolin, Zeolithe, Talk, Lehm, Asche, Kohlenstaub, Magnesiumsilikate, Dünger oder Mischungen der Substanzen. Hochdisperse pyrogene Kieselsäure wie sie unter dem Handelsnamen Aerosil von Evonik Degussa vertrieben wird ist bevorzugt.

Effektstoffe sind z.B. Polyzucker, polyphenolische Verbindungen wie z.B. hydrolisierbare Tannine oder einer Silizium-Sauerstoff beinhaltende Verbindungen oder eine Mischung von mindestens zwei darauf basierenden Effektstoffen. Der Effektstoff kann sowohl in fester Form (Pulver) als auch in gelöster Form mit einem Lösemittel zugegeben werden. Als Effektstoff wird im Rahmen der vorliegenden Erfindung insbesondere ein Stoff verstanden, der zur Geruchsinhibierung dient. Erfindungsgemäß werden hierunter Polyzucker verstanden unter denen der Fachmann solche aus der Gruppe von den geläufigen Stärken und deren Derivate, Cellulosen und deren Derivate, Cyclodextrine versteht. Wobei als Cyclodextrine vorzugsweise α-Cyclodextrin, β-Cyclodextrin, γ-Cyclodextrin oder Mischungen aus diesen Cyclodextrinen verstanden werden.

Als Silizium-Sauerstoff beinhaltende Verbindungen sind Zeolithe bevorzugt. Als Zeolithe können alle dem Fachmann bekannten synthetischen oder natürlichen Zeolithe eingesetzt werden. Bevorzugte natürliche Zeolithe sind Zeolithe aus der Natrolith-Gruppe Harmoton-Gruppe, der Mordenit-Gruppe, der Chabasit-Gruppe, der Faujasit-Gruppe (Sodalith-Gruppe) oder der Analcit-Gruppe. Beispiele für natürliche Zeolithe sind Analcim, Leucit, Pollucite, Wairakite, Bellbergite, Bikitaite, Boggsite, Brewsterite, Chabazit, Willhendersonite, Cowlesite, Dachiardite, Edingtonit, Epistilbit, Erionit, Faujasit, Ferrierite, Amicite, Garronite, Gismondine, Gobbinsite, Gmelinit, Gonnardite, Goosecreekit, Harmotom, Phillipsit, Wellsite, Clinoptilolit, Heulandit, Laumontit, Levyne, Mazzite, Merlinoite, Montesommaite, Mordenit, Mesolit, Natrolit, Scolecit, Offretite, Paranatrolite, Paulingite, Perlialite, Barrerite, Stilbit, Stellerit, Thomsonit, Tschernichite oder Yugawaralite. Bevorzugte synthetische Zeolithe sind Zeolith A, Zeolith X, Zeolith Y, Zeolith P oder das Produkt ABSCENTS®.

Als Kationen enthalten die in dem erfindungsgemäßen Verfahren einsetzbaren Zeolithe vorzugsweise Alkalimetall-Kationen wie Li⁺, Na⁺, K⁺, Rb⁺, Cs⁺ oder Fr⁺ und/oder Erdalkalimetall-Kationen wie Mg²⁺, Ca²⁺, Sr²⁺ oder Ba²⁺.

Als Zeolithe können Zeolithe des sogenannten "mittleren" Typs eingesetzt werden, bei denen das SiO₂/AlO₂-Verhältnis kleiner als 10 ist, besonders bevorzugt liegt das SiO₂/AlO₂-Verhältnis dieser Zeolithe in einem Bereich von 2 bis 10. Neben diesen "mittleren" Zeolithen können weiterhin Zeolithe des "hohen" Typs eingesetzt werden, zu denen beispielsweise die bekannten "Molekularsieb"-Zeolithe des Typs ZSM sowie beta-Zeolith gehören. Diese "hohen" Zeolithe sind vorzugsweise durch ein SiO₂/AlO₂-Verhältnis von mindestens 35, besonders bevorzugt von einem SiO₂/AlO₂-Verhältnis in einem Bereich von 200 bis 500 gekennzeichnet.

Vorzugsweise werden die Zeolithe als Partikel mit einer mittleren Partikelgröße in einem Bereich von 1 bis 500 µm, besonders bevorzugt in einem Bereich von 2 bis 200 µm und darüber hinaus bevorzugt in einem Bereich von 5 bis 100 µm eingesetzt.

Die Effektstoffe können in dem erfindungsgemäßen Verfahren vorzugsweise in einer Menge in einem Bereich von 0,1 bis 50 Gew.-%, besonders bevorzugt in einem Bereich von 1 bis 40 Gew.-% und darüber hinaus bevorzugt in einer Menge in einem Bereich von 5 bis 30 Gew.-%, jeweils bezogen auf das Gewicht der wasserabsorbierenden Polymerteilchen, eingesetzt werden.

Als keimhemmende Mittel sind grundsätzlich alle gegen grampositive Bakterien wirksamen Stoffe bevorzugt, wie z. B. 4-Hydroxybenzoesäure und ihre Salze und Ester, N-(4-Chlorphenyl)-N'-(3,4dichlorphenyl)harnstoff, 2,4,4'-Trichlor-2'-hydroxydiphenylether (Triclosan), 4-Chlor-3,5-dimethylphenol, 2,2'- Methylen-bis(6-brom-4-chlorphenol), 3-Methyl-4-(1-methylethyl)phenol, 2-Benzyl-4-chlorphenol, 3-(4- Chlorphenoxy)-1,2-propandiol, 3-lod-2-propinylbutylcarbamat, Chlorhexidin, 3,4,4'-Trichlorcarbonilid (TTC), antibakterielle Riechstoffe, Thymol, Thymianöl, Eugenol, Nelkenöl, Menthol, Minzöl, Famesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen TM CAT, Cognis GmbH, Düsseldorf/Deutschland). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbnonsäuren und deren Ester wie beispielsweise Citronensäure, Äpfelsäure, Weinsäure oder Weinsäurediethylester, sowie Zinkglycinat.

Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, dass dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z. B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z. B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z. B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z. B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, alpha -Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, beta-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

Antitranspirantien reduzieren durch Beeinflussung der Aktivität der ekkrinen Schweißdrüsen die Schweißbildung, und wirken somit Achselnässe und Körpergeruch entgegen. Als adstringierende Antitranspirant-Wirkstoffe eignen sich vor allem Salze des Aluminiums, Zirkoniums oder des Zinks. Solche geeigneten antihydrotisch wirksamen Wirkstoffe sind z. B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium- Zirkonium-Trichlorohydrat, Aluminium-Zirkonium-Tetrachlorohydrat, Aluminium-Zirkonium-Pentachlorohydrat und deren Komplexverbindungen z. B. mit Aminosäuren wie Glycin.

Zum Mischen bzw. Besprühen sind im Rahmen dieser Erfindung grundsätzlich alle Vorrichtungen geeignet, die eine homogene Verteilung einer Lösung, Pulver, Suspension oder Dispersion auf oder mit den Hydrogel-Polymerteilchen oder wasserabsorbierenden Polymeren erlauben. Beispiele sind Lödige Mischer (hergestellt durch die Firma *Gebrüder Lödige Maschinenbau GmbH*), Gericke Multi-Flux Mischer (hergestellt durch die Firma *Gericke GmbH*), DRAIS-Mischer (hergestellt durch die Firma *DRAIS GmbH* Spezialmaschinenfabrik Mannheim), Hosokawa Mischer (*Hosokawa Mokron Co., Ltd.*), Ruberg Mischer (hergestellt durch die Firma *Gebr. Ruberg GmbH* & *CO.KG Nieheim*), Hüttlin Coater (hergestellt durch die Firma *BWI Hüttlin GmbH Steinen*), Fließbetttrockner oder Sprühgranulatoren von AMMAG (hergestellt durch die Firma *AMMAG Gunskirchen, Österreich*) oder Heinen (hergestellt durch die *Firma A. Heinen AG Anlagenbau Varel*), Patterson-Kelly-Mischer, NARA-Schaufelmischer, Schneckenmischer, Tellermischer, Wirbelschichttrockner oder Schugi-Mischer. Für das in Kontakt bringen in einer Wirbelschicht können alle dem Fachmann bekannten und geeignet erscheinenden Wirbelschichtverfahren angewandt werden. Beispielsweise kann ein Wirbelschichtcoater eingesetzt werden.

Erfindungsgemäß ist es besonders vorteilhaft, die Polymerpartikel, die beim Oberflächenvernetzungsschritt resultieren, entspricht Schritt (vi), mit Hilfe eines Becherwerks und/oder Rohrkettenförderers, insbesondere Becherwerks, dem Kühlungsschritt (vii), d.h. der betreffenden Kühlapparatur zuzuführen.

Erfindungsgemäß ist es weiterhin besonders vorteilhaft, die Polymerpartikel, die beim Kühlschritt resultieren, entspricht Schritt (vii), mit Hilfe eines Becherwerks und/oder Rohrkettenförderers, insbesondere Becherwerks, dem optionalen Nachbehandlungsschritt zuzuführen.

Die optionale Nachbehandlung nach der Oberflächennachvernetzung wird vorzugsweise in geeigneten Mischgeräten oder in Kontakttrocknern, besonders bevorzugt Schaufeltrocknern, ganz besonders bevorzugt Scheibentrocknern, durchgeführt. Geeignete Geräte sind beispielsweise Hosokawa Bepex® Horizontal Paddle Dryer (Hosokawa Micron GmbH; Leingarten; Deutschland), Hosokawa Bepex® Disc Dryer (Hosokawa Micron GmbH; Leingarten; Deutschland) und Nara Paddle Dryer (NARA Machinery Europe; Frechen; Deutschland). Überdies können auch Wirbelschichttrockner eingesetzt werden.

Der optionale Nachbehandlungsschritt umfasst vorzugsweise die Behandlung der oberflächenvernetzten Polymerpartikel durch
i) Beaufschlagung (vorzugsweise Beschichtung) der Polymerpartikel, vorzugsweise mit mindestens einem Salz aus einem polyvalenten Metallkation und einem nichtkomplexierenden Säuranion,
   und/oder mit einer Verbindung mit Entstaubungsvermögen, wie vorzugsweise Polyol oder Polyethylenglykol,
   und/oder mit geruchshemmenden oder geruchsvermindernden Substanzen, vorzugsweise Tannin-haltigen wässrigen Lösungen,
   wobei die Beschichtungssubstanzen jeweils vorzugsweise aus wässriger Lösung aufgebracht werden, insbesondere durch Aufsprühen,
   und/oder
ii) Erhöhung des Feuchtgehalts der Polymerpartikel, vorzugsweise um 1 bis 150 Gew.-% und
iii) optional Trocknung nach der Erhöhung des Feuchtgehalts.

Unter einem "Tannin" im Sinne der vorliegenden Erfindung werden im Allgemeinen natürlich vorkommende Polyphenole verstanden. Grundsätzlich können erfindungsgemäss sogenannte "kondensierte Tannine" oder aber "hydrolysierbare Tannine" eingesetzt werden, wobei der Einsatz hydrolyierbarer Tannine besonders bevorzugt und der Einsatz hydrolysierbarer Gallotannine am meisten bevorzugt ist. Solche Verbindungen sind an sich bekannt und werden z.B. in der deutschen Patentanmeldung DE102007045724A1 beschrieben, auf die Hiermit Bezug genommen wird. Unter "kondensierten Tanninen" werden vorzugsweise Tannine verstanden, welche Oligomere oder Polymere aus Flavonoid-Einheiten darstellen, welche über C-C-Bindungen miteinander verknüpft sind. Solche kondensierten Tannine umfassen üblicherweise 2 bis 50 Flavonoid-Einheiten auf, können aber auch aus mehr als 50 Flavonoid-Einheiten bestehen. Als Flavonoid-Einheiten kommen dabei insbesondere Catechin und Epicatechin in Frage. Unter "hydrolysierbaren Tanninen" werden vorzugsweise Tannine verstanden, die aus einem Polyol, beispielsweise einem Kohlenhydrat, als Kern bestehen, wobei an die OH-Gruppen dieses Kernmoleküls Gallsäure über Esterbindungen angebunden sind. Solche auf Gallsäure basierenden, hydrolysierbaren Tannin werden daher häufig auch als "Gallotannine" bezeichnet. Neben Gallsäure können die hydrolysierbaren Tannine aber auch auf Ellaginsäure basieren. Solche hydrolysierbaren Tannine werden häufig auch als "Ellagitannine" bezeichnet.

Anschließend, also nach der optionalen Nachbehandlung, können die oberflächennachvernetzten und nachbehandelten Polymerpartikel erneut klassiert werden, wobei zu kleine und/oder zu große Polymerpartikel abgetrennt und in das Verfahren rückgeführt werden. Der Transport, insbesondere umfassend vertikale Förderung, der oberflächennachvernetzten und optional nachbehandelten Polymerpartikel zu der Siebvorrichtung für das optionale erneute Klassieren wird vorzugsweise mit Hilfe von Becherwerk und/oder Rohrkettenförderer bewirkt.

Anschließend, also nach dem optionalen Klassieren, können die Polymerpartikel dann in die Endproduktsilos oder Silofahrzeuge verbracht werden, wobei der Transport, insbesondere umfassend vertikale Förderung, der betreffenden Polymerpartikel in die Endproduktsilos oder Silofahrzeuge vorzugsweise mit Hilfe von Becherwerk und/oder Rohrkettenförderer, insbesondere mit Hilfe von Rohrkettenförderer bewirkt wird.

Ein Verfahren zur Herstellung von wasserabsorbierenden oberflächenvernetzten Polymerpartikeln umfassend
(i) die Polymerisation einer Monomerlösung oder -suspension, enthaltend
   a) mindestens ein ethylenisch ungesättigtes, säuregruppentragendes Monomer, das zumindest teilweise neutralisiert sein kann,
   b) mindestens einen Vernetzer,
   c) mindestens einen Initiator,
   d) optional ein oder mehrere mit den unter a) genannten Monomeren copolymerisierbare ethylenisch ungesättigte Monomere und
   e) optional ein oder mehrere wasserlösliche Polymere,
      um ein wasserunlösliches Polymergel zu bilden,
(ii) Zerkleinern des Polymergels,
(iia) Auflockerung des Polymergels, vorzugsweise in einer Auflockerungseinrichtung, welche insbesondere eine rotierende Trommel ist,
(iii) Trocknen des Polymergels,
(iv) Mahlen des Polymergels zu Polymerpartikeln,
(v) Klassieren der Polymerpartikel,
(vi) Oberflächennachvernetzung der klassierten Polymerpartikel,
(vii) Kühlung und Nachbehandlung des oberflächenvernetzten Polymers,
(viii) Klassieren der Polymerpartikel,
wobei das Verfahren Förderschritte für das darin anfallenden teilchenförmige Gut umfasst, wobei zur (insbesondere vertikalen) Förderung des teilchenförmigen Gutes nach Schritt (vi) jeweils im Wesentlichen auf Rohrkettenförderer zurückgegriffen wird,
stellt eine bevorzugte Ausführungsform der Erfindung dar. Dabei wird in der vorgenannten Ausführungsform insbesondere zwischen den Schritten (vi), (vii) und (viii) zur (insbesondere vertikalen) Förderung im Wesentlichen auf Becherwerk zurückgegriffen und nach Schritt (viii), betreffend den Transport in die Endsilos, inbesondere umfassend vertikale Förderung, im Wesentlichen auf Rohrkettenförderer zurückgegriffen.

"Im Wesentlichen" bedeutet hierbei, dass zumindest > 50%, vorzugsweise > 60%, vorteilhafterweise > 70%, in weiter vorteilhafter Weise > 80%, in noch weiter vorteilhafter Weise > 85%, in wiederum vorteilhafterer Weise > 90% und insbesondere > 95%, z.B. 100%, der zurückzulegenden (insbesondere vertikalen) Transportstrecke mit Becherwerk und/oder Rohrkettenförderer bewältigt wird.

Insbesondere wird zwischen den Schritten (vi), (vii) und (viii) und nach Schritt (viii) im Wesentlichen auf pneumatische Fördermaßnahmen verzichtet, insbesondere bei vertikaler Förderung.

"Im Wesentlichen auf pneumatische Fördermaßnahmen zu verzichten" bedeutet hierbei, dass zumindest > 50%, vorzugsweise > 60%, vorteilhafterweise > 70%, in weiter vorteilhafter Weise > 80%, in noch weiter vorteilhafter Weise > 85%, in wiederum vorteilhafterer Weise > 90% und insbesondere > 95%, z.B. 100 %, der zurückzulegenden (insbesondere vertikalen) Transportstrecke ohne Zuhilfenahme pneumatischer Fördertechnik bewältigt wird.

Eine vertikale Transportstrecke im Sinne dieser Erfindung ist eine Transportstrecke, die einen Höhenunterschied überwindet, insbesondere einen Höhenunterschied von mindestens einem Meter, vorzugsweise von mindestens zwei Metern. Eine Obergrenze kann z.B. bei 25 Metern oder z.B. bei 10 Metern oder z.B. bei 5 Metern liegen.

Die mit dem erfindungsgemäßen Verfahren erhältlichen wasserabsorbierenden Polymerpartikel werden üblicherweise als Superabsorber bezeichnet.

Die wasserabsorbierenden Polymerpartikel weisen eine Zentrifugenretentionskapazität (CRC) von vorteilhafterweise mindestens 15 g/g, vorzugsweise mindestens 20 g/g, bevorzugt mindestens 22 g/g, besonders bevorzugt mindestens 24 g/g, ganz besonders bevorzugt mindestens 26 g/g, auf. Ein bevorzugter Bereich für die Zentrifugenretentionskapazität (CRC) liegt z.B. zwischen 24-32 g/g. Die Zentrifugenretentionskapazität (CRC) der wasserabsorbierenden Polymerpartikel beträgt üblicherweise weniger als 60 g/g. Die Zentrifugenretentionskapazität (CRC) wird gemäß der von der EDANA (European Disposables and Nonwovens Association) empfohlenen Testmethode Nr. 441.2-02 "Centrifuge retention capacity" bestimmt.

Die wasserabsorbierenden Polymerpartikel weisen eine Absorption gegen einen Druck von 4,83 kPa (entspricht dem AAP-Wert) von vorteilhafterweise mindestens 10 g/g, vorzugsweise mindestens 15 g/g, bevorzugt mindestens 20 g/g, besonders bevorzugt mindestens 22 g/g, ganz besonders bevorzugt mindestens 23 g/g, weiter bevorzugt mindestens 24 g/g auf. Die Absorption gegen einen Druck von 4,83 kPa der wasserabsorbierenden Polymerpartikel beträgt üblicherweise weniger als 30 g/g. Die Absorption gegen einen Druck von 4,83 kPa wird gemäß der von der EDANA (European Disposables and Nonwovens Association) empfohlenen Testmethode Nr. 442.2-02 "Absorption under pressure" bestimmt. Der AAP-Wert im Sinne dieser Erfindung ist die Absorption gegen einen Druck von 4,83 kPa, bestimmt gemäß der von der EDANA (European Disposables and Nonwovens Association) empfohlenen Testmethode Nr. 442.2-02 "Absorption under pressure".

Die wasserabsorbierenden Polymerpartikel weisen eine Permeabilität (SFC) von vorteilhafterweise mindestens 50 x 10⁻⁷ cm³s/g, vorzugsweise mindestens 60 x 10⁻⁷ cm³s/g, bevorzugt mindestens 70 x 10⁻⁷ cm³s/g, besonders bevorzugt mindestens 80 x 10⁻⁷ cm³s/g, ganz besonders bevorzugt mindestens 90 x 10⁻⁷ cm³s/g, auf. Permeabilität (SFC) der wasserabsorbierenden Polymerpartikel beträgt üblicherweise weniger als 250 x 10⁻⁷ cm³s/g. Die Bestimmung der Permeabilität (SFC) erfolgt durch die Messung der sogenannten "Saline Flow Conductivity-SFC" gemäß dem in der WO95/26209 A1 beschriebenen Testverfahren. Die Einwaage des Superabsorbermaterials beträgt 1,5 g anstatt 0,9 g. Der SFC-Wert ist in der vorliegenden Erfindung immer bezogen auf 1,5g des Superabsorbermaterials.

Die Gelbettpermeabilität (GBP) einer gequollenen Gelschicht kann insbesondere unter Druckbelastung von 0,3 psi (2070 Pa), wie in US 2005/02567575 beschrieben (dort Absätze [0061] und [0075]), als Gel-Bed-Permeability einer gequollenen Gelschicht aus wasserabsorbierenden Polymerpartikeln bestimmt werden.

Vorzugsweise weisen die wasserabsorbierende Polymerpartikel
(a) eine Zentrifugenretentionskapazität (CRC) von mindestens 24 g/g,
(b) eine Quellgeschwindigkeit (FSR) von mindestens 0,15 g/gs.
(c) eine Permeabilität (SFC) von mindestens 50 x 10⁻⁷ cm³s/g,
(d) eine Absorption unter einem Druck von 4,83 kPa von mindestens 15 g/g,
(e) eine Oberflächenspannung vorzugsweise über 50 mN/m,
auf.

Die Bestimmung der Oberflächenspannung erfolgt durch die Messung gemäß dem in EP 1 493 453 A1 beschriebenen Testverfahren gemäß Seite 12 Absatz [0105] bis [0111], wobei insbesondere ein Tensiometer der Marke Kruss K11 mit einer Wilhelmyplatte verwendet wird.

Vorzugsweise beträgt der Anteil an wasserabsorbierenden Polymerpartikeln mit einer Partikelgröße von mindestens 150 µm mindestens 90 Gew.-%, besonders bevorzugt mindesten 95 Gew.-%, ganz besonders bevorzugt mindestens 98 Gew.-% beträgt und Anteil an wasserabsorbierenden Polymerpartikeln mit einer Partikelgröße von höchstens 850 µm, beträgt vorzugsweise mindestens 90 Gew.-%, besonders bevorzugt mindesten 95 Gew.-%, ganz besonders bevorzugt mindestens 98 Gew.-%, jeweils bezogen auf die Gesamtmenge der wasserabsorbierenden Polymerpartikel.

Ein weiterer Gegenstand der vorliegenden Offenbarung sind Hygieneartikel, enthaltend erfindungsgemäß erhältliche wasserabsorbierende Polymerpartikel, insbesondere Hygieneartikel zur Damenhygiene, Hygieneartikel für leichte und schwere Inkontinenz, Windeln oder Kleintierstreu.

Die Herstellung der Hygieneartikel wird in der Monographie "Modern Superabsorbent Polymer Technology", F.L. Buchholz und AT. Graham, Wiley-VCH, 1998, Seiten 252 bis 258, beschrieben. Dort wird auch die Herstellung wasserabsorbierender Polymerpartikel beschrieben.

Die Hygieneartikel enthalten üblicherweise eine wasserundurchlässige Rückseite, eine wasserdurchlässige Oberseite und dazwischen einen absorbierenden Kern aus den erfindungsgemäßen wasserabsorbierenden Polymerpartikeln und Fasern, vorzugsweise Cellulose. Der Anteil der erfindungsgemäßen wasserabsorbierenden Polymerpartikel im absorbierenden Kern beträgt vorzugsweise 20 bis 100 Gew.-%, bevorzugt 50 bis 100 Gew.-%.

Ein weiterer Gegenstand dieser Offenbarung ist ein Verbund, beinhaltend die die durch die erfindungsgemäßen Verfahren erhältlichen wasserabsorbierenden Polymerpartikel und ein Substrat. Es ist dabei bevorzugt, dass die wasserabsorbierenden Polymere und das Substrat miteinander fest verbunden sind. Als Substrate sind Folien aus Polymeren, wie beispielsweise aus Polyethylen, Polypropylen oder Polyamid, Metalle, Vliese, Fluff, Tissues, Gewebe, natürliche oder synthetische Fasern, oder Schäume bevorzugt. Weiterhin ist es erfindungsgemäß bevorzugt, dass der Verbund mindestens einen Bereich umfasst, welcher wasserabsorbierenden Polymerpartikel in einer Menge im Bereich von etwa 15 bis 100 Gew.-%, vorzugsweise etwa 30 bis 100 Gew.-%, besonders bevorzugt von etwa 50 bis 99,99 Gew.-%, ferner bevorzugt von etwa 60 bis 99,99 Gew.-% und darüber hinaus bevorzugt von etwa 70 bis 99 Gew.-%, jeweils bezogen auf das Gesamtgewicht des betreffenden Bereichs des Verbundes, beinhaltet, wobei dieser Bereich vorzugsweise eine Größe von mindestens 0,01 cm³, vorzugsweise mindestens 0,1 cm³ und am meisten bevorzugt mindestens 0,5 cm³ aufweist.

Ein weiterer Gegenstand dieser Offenbarung ist ein Verfahren zur Herstellung eines Verbundes, wobei die erfindungsgemäß erhältliche wasserabsorbierende Polymerpartikel bzw. die durch das erfindungsgemäße Verfahren erhältlichen Superabsorber und ein Substrat und gegebenenfalls ein Zusatzstoff miteinander in Kontakt gebracht werden. Als Substrate werden vorzugsweise diejenigen Substrate eingesetzt, die bereits vorstehend im Zusammenhang mit dem erfindungsgemäßen Verbund genannt wurden.

Ein weiterer Gegenstand dieser Offenbarung ist ein Verbund erhältlich nach dem vorstehend beschriebenen Verfahren, wobei dieser Verbund vorzugsweise die gleichen Eigenschaften aufweist wie der vorstehend beschriebene, erfindungsgemäße Verbund.

Ein weiterer Gegenstand dieser Offenbarung sind chemische Produkte beinhaltend die erfindungsgemäß erhältlichen wasserabsorbierenden Polymerpartikel oder einen erfindungsgemäßen Verbund. Bevorzugte chemische Produkte sind insbesondere Schäume, Formkörper, Fasern, Folien, Filme, Kabel, Dichtungsmaterialien, flüssigkeitsaufnehmende Hygieneartikel, insbesondere Windeln und Damenbinden, Träger für pflanzen- oder pilzwachstumsregulierende Mittel oder Pflanzenschutzwirkstoffe, Zusätze für Baustoffe, Verpackungsmaterialien oder Bodenzusätze.

Auch die Verwendung der erfindungsgemäß erhältlichen wasserabsorbierenden Polymerpartikel oder des Verbundes in chemischen Produkten, vorzugsweise in den vorstehend genannten chemischen Produkten, insbesondere in Hygieneartikeln wie Windeln oder Damenbinden, sowie die Verwendung der wasserabsorbierenden Polymerpartikel als Träger für pflanzen- oder pilzwachstumsregulierende Mittel oder Pflanzenschutzwirkstoffe stellen einen weiteren Gegenstand dieser Offenbarung dar. Bei der Verwendung als Träger für pflanzen- oder pilzwachstumsregulierende Mittel oder Pflanzenschutzwirkstoffe ist es bevorzugt, dass die pflanzen- oder pilzwachstumsregulierende Mittel oder Pflanzenschutzwirkstoffe über einen durch den Träger kontrollierten Zeitraum abgegeben werden können.

### Testmethoden

Sofern nicht anders angegeben, werden die genannten Messungen insbesondere nach ERT-Verfahren durchgeführt. "ERT" steht für EDANA Recommended Test und "EDANA" für European Disposable and Nonwoven Association. Diese ERT-Verfahren und andere Testmethoden wurden schon weiter oben angegeben. Die EDANA-Testmethoden sind beispielsweise erhältlich bei der EDANA, Avenue Eugene Plasky 157, B-1030 Brüssel, Belgien.

Alle Testmethoden werden grundsätzlich, wenn nichts anderes angegeben ist, bei einer Umgebungstemperatur von 23±2 °C und einer relativen Luftfeuchte von 50±10 % durchgeführt werden.

Die im Rahmen dieser Erfindung genannten Methoden können zur Charakterisierung der bei dem Verfahren anfallenden Superabsorber eingesetzt werden, wobei das erfindungsgemäße Verfahren sich grundsätzlich auf alle genannten Parameter günstig auswirkt, insbesondere aber das Erzielen besonders guter AAP-Werte ermöglicht.

### Beispiel

Das erfindungsgemäße Verfahren kann grundsätzlich in alle bestehenden, insbesondere großtechnischen Prozesse zur Superabsorberherstellung implementiert werden.

### Allgemeines Herstellverfahren

300 kg Acrylsäure wurde mit 429,1 kg H₂O; 1,2 kg Allyloxypolyethylenglykolacrylsäureester und 1,2 kg Polyethylenglykol-300-di- acrylat gemischt und auf 10°C gekühlt. Danach wurden unter Kühlen insgesamt 233,1 kg 50%ige Natronlauge so langsam zugesetzt, dass die Temperatur 30°C nicht überstieg. Anschließend wurde die Lösung bei 20°C mit Stickstoff gespült und dabei weiter abgekühlt. Bei Erreichen der Starttemperatur von 4°C wurden die Initiatorlösungen (0,1 kg 2,2'-Azobis-2-amidinopropan-dihydrochlorid in 10 kg H₂O; 0,15 kg Natriumperoxydisulfat in 10 kg H₂O; 0,1 kg 30%ige Wasserstoffperoxidlösung in 1 kg H₂O und 0,01 kg Ascorbinsäure in 2 kg Wasser) zugesetzt. Die Polymerisation wurde auf einem Endlosband mit einer Verweilzeit von ca. 40 Minuten durchgeführt.

Das entstandene Gel wurde zerkleinert und bei 150 - 180°C 60 Minuten getrocknet. Das getrocknete Polymerisat wurde grob zerstoßen, gemahlen und kontinuierlich zu einem Pulver mit einer Partikelgröße von 150 bis 850 µm abgesiebt.

Dieses Fraktion wurde zur Oberflächennachvernetzung mit 2 % einer Lösung aus 1 Teil Ethylencarbonat und 1 Teil Wasser kontinuierlich in einem Mischer beschichtet und in einem Paddeltrockner auf 185°C erhitzt (Verweilzeit ca. 40 Min.).

Das so erhaltene Produkt wurde nach dem Abkühlen erneut klassiert, wobei die Fraktion mit einer Partikelgröße von 150 bis 850 µm als Verfahrensendprodukt betrachtet wurde. Das resultierende teilchenförmige, oberflächenvernetzte Verfahrensendprodukt wurde dann jeweils in einen Silo zur Lagerung eingefüllt.

### Förderschritte

Bei dem zuvor beschriebenen Herstellverfahren waren Förderschritte notwendig, und zwar musste u.a. das teilchenförmige Zwischenprodukt nach dem 1. Klassieren zu dem Oberflächenvernetzungsschritt befördert werden (entspricht Förderschritt a), und weiterhin wurde u.a. auch das teilchenförmige, oberflächenvernetzte Endprodukt zum Schluß in einen Lagersilo befördert (entspricht Förderschritt c).

### Variante a

Bei der Variante a wurden im Rahmen der zuvor genannten Förderschritte a) und c) im Einklang mit der vorliegenden Erfindung auf Rohrkettenförderer im Schritt c) und Becherwerk im Schritt a) zurückgegriffen. Das dabei im Lagersilo anfallende Endprodukt wird als Endprodukt a bezeichnet.

### Variante b

Bei der Variante b wurden die zuvor genannten Förderschritte in üblicher Weise unter Einsatz pneumatischer Fördertechnik durchgeführt. Das dabei im Lagersilo anfallende Endprodukt wird als Endprodukt b bezeichnet. Endprodukt a und b unterscheiden sich also in der Herstellung alleine dadurch, dass jeweils unterschiedliche Förderschritte gemäß den gerade genannten Varianten a oder b eingesetzt worden sind.

### Ergebnis:

Nach jeweils fünffacher Wiederholung der jeweiligen Herstellung konnte gefunden werden, dass der AAP-Wert (genauer AAP 4,83 kPa) des Endproduktes a durchschnittlich um 0,9 g/g höher lag als der AAP-Wert des Endproduktes b. Das erfindungsgemäße Verfahren ermöglicht somit die Bereitstellung von wasserabsorbierenden oberflächenvernetzten Polymerpartikeln mit möglichst gutem AAP-Wert. Die Partikelgrößenverteilung, Permeabilität (SFC) sowie Gelbettpermeabilität (GBP) wurden bei Variante a im Gegensatz zu Variante b nicht beeinträchtigt.

## Patentansprüche

1. Verfahren zur Herstellung wasserabsorbierender Polymerpartikel,
umfassend
(i) die Polymerisation einer Monomerlösung oder -suspension, enthaltend
a) mindestens ein ethylenisch ungesättigtes, säuregruppentragendes Monomer, das zumindest teilweise neutralisiert sein kann,
b) mindestens einen Vernetzer,
c) mindestens einen Initiator,
d) optional ein oder mehrere mit den unter a) genannten Monomeren copolymerisierbare ethylenisch ungesättigte Monomere und
e) optional ein oder mehrere wasserlösliche Polymere,
um ein wasserunlösliches Polymergel zu bilden,
(ii) optional Zerkleinern des Polymergels,
(iia) optional Auflockerung des Polymergels in einer Auflockerungseinrichtung, welche vorzugsweise eine rotierende Trommel ist,
(iii) Trocknen des Polymergels,
(iv) Mahlen des Polymergels zu Polymerpartikeln,
(v) Klassieren der Polymerpartikel,
(vi) Oberflächennachvernetzung der klassierten Polymerpartikel,
(vii) Kühlung und optional Nachbehandlung der oberflächenvernetzten Polymerpartikel,
wobei das Verfahren Förderschritte für das darin anfallende teilchenförmige Gut umfasst,
und wobei bei wenigstens einem der Förderschritte eine Fördermaschine aus der Gruppe der mechanischen Stetigförderer mit Zugmittel eingesetzt wird,
**dadurch gekennzeichnet, dass** als Fördermaschine ein Rohrkettenförderer eingesetzt wird, wobei der Rohrkettenförderer ein Rohr als Tragmittel, eine Kette mit daran befestigten Stau- und Mitnehmerscheiben als Zugmittel sowie eine Antriebsstation aufweist.

2. Verfahren zur Herstellung von wasserabsorbierenden Polymerpartikeln nach Anspruch 1, **dadurch gekennzeichnet, dass** wenigstens bei einem der Förderschritte (a), (b), vorzugsweise zumindest bei einem Förderschritt (b), insbesondere bei beiden Förderschritten (a), (b) Fördermaschinen aus der Gruppe der mechanischen Stetigförderer mit Zugmittel eingesetzt werden,
wobei der Förderschritt (a) vor dem Oberflächennachvernetzungsschritt (vi) liegt und der Förderschritt (b) nach dem Oberflächennachvernetzungsschritt (vi) liegt.

3. Verfahren nach einem der Ansprüche 1 oder 2, **dadurch gekennzeichnet, dass** bei dem Förderschritt (c) Fördermaschinen aus der Gruppe der mechanischen Stetigförderer mit Zugmittel eingesetzt werden,
wobei der Förderschritt (c) den Transport des fertigen Superabsorberendproduktes, also der hergestellten wasserabsorbierenden Polymerpartikel, in die Endproduktsilos oder Silofahrzeuge hinein betrifft.

4. Verfahren nach einem der Ansprüche 2 oder 3, **dadurch gekennzeichnet, dass** zumindest der Förderschritt (b), vorzugsweise zumindest die Förderschritte (b) und (c), vorteilhafterweise die Förderschritte (a), (b) und (c), insbesondere sämtliche Förderschritte unter Einsatz von Fördermaschinen aus der Gruppe der mechanischen Stetigförderer mit Zugmittel erfolgt.

5. Verfahren nach einem der Ansprüche 1 bis 4, **dadurch gekennzeichnet, dass** weiterhin zumindest in einem der Förderschritte zusätzlich ein Schneckenförderer eingesetzt wird.

6. Verfahren nach einem der Ansprüche 1 bis 5, **dadurch gekennzeichnet, dass** bei der Polymerisation der Monomerlösung oder -suspension ein Blähmittel eingesetzt wird, so dass ein geschäumtes wasserunlösliches Polymergel gebildet wird.

7. Verfahren nach einem der Ansprüche 1 bis6, **dadurch gekennzeichnet, dass** als Blähmittel ein Gas, wie insbesondere CO2 eingesetzt wird, oder eine Verbindung mit Gasfreisetzungsvermögen, wie insbesondere Carbonatsalze.

8. Verfahren nach einem der Ansprüche 1 bis 7, **dadurch gekennzeichnet, dass** die Monomerlösung oder -suspension mindestens ein Tensid, vorzugsweise ein ethylenisch ungesättigtes Tensid enthält.

9. Verfahren nach Anspruch 8, **dadurch gekennzeichnet, dass** das Tensid in einer Menge von 0,01 bis 5 Gew.-%, bevorzugt 0,015 bis 2 Gew.-%, weiter bevorzugt 0,02 bis 1 Gew.-%, insbesondere 0,02 bis 0,5 Gew.-% in der Monomerlösung oder -suspension enthalten ist.

10. Verfahren gemäß einem der Ansprüche 1 bis 9 , **dadurch gekennzeichnet, dass** das Monomer a) Acrylsäure ist, sowie dass zur Oberflächennachvernetzung in Schritt vi) eine Verbindung eingesetzt wird, die mit mindestens zwei Säuregruppen der Polymerpartikel kovalente Bindungen ausbilden kann, sowie dass die Polymerpartikel nach Schritt v) beschichtet werden, vorzugsweise mit mehrwertigen Metallkationen, wobei die mehrwertigen Metallkationen insbesondere Aluminiumkationen sind.

## Claims

1. Process for producing water-absorbing polymer particles,
comprising
(i) the polymerization of a monomer solution or suspension comprising
a) at least one ethylenically unsaturated monomer which bears acid groups and may have been at least partly neutralized,
b) at least one crosslinker,
c) at least one initiator,
d) optionally one or more ethylenically unsaturated monomers copolymerizable with the monomers mentioned in a) and
e) optionally one or more water-soluble polymers,
in order to form a water-insoluble polymer gel,
(ii) optionally comminuting the polymer gel,
(iia) optionally breaking up the polymer gel in a breakup unit which is preferably a rotating drum,
(iii) drying the polymer gel,
(iv) grinding the polymer gel to polymer particles,
(v) classifying the polymer particles,
(vi) surface postcrosslinking the classified polymer particles,
(vii) cooling and optionally after treating the surface crosslinked polymer particles,
the process comprising conveying steps for the particulate material that arises, and a conveying machine from the group of the mechanical continuous conveyors with traction mechanism being used in at least one of the conveying steps, **characterized in that** the conveying machine used is a tubular drag conveyor, wherein the tubular drag conveyor comprises a tube as carrier means, a chain with backup and entrainment disks as traction mechanism secured thereto, and a drive station.

2. Process for producing water-absorbing polymer particles according to Claim 1, **characterized in that** conveying machines from the group of the mechanical continuous conveyors with traction mechanism are used in at least one of the conveying steps (a) and (b), preferably at least in a conveying step (b), especially in both conveying steps (a) and (b),
where the conveying step (a) precedes the surface postcrosslinking step (vi) and the conveying step (b) follows the surface postcrosslinking step (vi).

3. Process according to either of Claims 1 and 2, **characterized in that** conveying machines from the group of the mechanical continuous conveyors with traction mechanism are used in the conveying step (c),
where the conveying step (c) relates to the transport of the finished superabsorbent end product, i.e. of the water-absorbing polymer particles produced, into the end product silos or silo vehicles.

4. Process according to either of Claims 2 and 3, **characterized in that** at least conveying step (b), preferably at least conveying steps (b) and (c), advantageously conveying steps (a), (b) and (c), especially all the conveying steps, are effected using conveying machines from the group of the mechanical continuous conveyors with traction mechanism.

5. Process according to any of Claims 1 to 4, **characterized in that** a screw conveyor is additionally used at least in one of the conveying steps.

6. Process according to any of Claims 1 to 5, **characterized in that** a blowing agent is used in the polymerization of the monomer solution or suspension, such that a foamed water-insoluble polymer gel is formed.

7. Process according to any of Claims 1 to 6, **characterized in that** the blowing agent used is a gas, such as CO2 in particular, or a compound having ability to release gas, such as carbonate salts in particular.

8. Process according to any of Claims 1 to 7, **characterized in that** the monomer solution or suspension comprises at least one surfactant, preferably an ethylenically unsaturated surfactant.

9. Process according to Claim 8, **characterized in that** the surfactant is present in the monomer solution or suspension in an amount of 0.01% to 5% by weight, preferably 0.015% to 2% by weight, further preferably 0.02% to 1% by weight, especially 0.02% to 0.5% by weight.

10. Process according to any of Claims 1 to 9, **characterized in that** the monomer a) is acrylic acid, and **in that** a compound which can form covalent bonds with at least two acid groups of the polymer particles is used for surface postcrosslinking in step vi), and **in that** the polymer particles are coated after step v), preferably with polyvalent metal cations, the polyvalent metal cations especially being aluminum cations.

## Revendications

1. Procédé pour la fabrication de particules polymères absorbant l'eau, comprenant
(i) la polymérisation d'une solution ou d'une suspension de monomères, contenant
a) au moins un monomère éthyléniquement insaturé, portant des groupes acides, qui peut être au moins partiellement neutralisé,
b) au moins un réticulant,
c) au moins un initiateur,
d) éventuellement un ou plusieurs monomères éthyléniquement insaturés copolymérisables avec les monomères cités en a) et
e) éventuellement un ou plusieurs polymères solubles dans l'eau pour former un gel polymère insoluble dans l'eau,
(ii) éventuellement la fragmentation du gel polymère,
(iia) éventuellement la désagrégation du gel polymère dans un dispositif de désagrégation qui est de préférence un tambour rotatif,
(iii) le séchage du gel polymère,
(iv) le broyage du gel polymère en particules polymères,
(v) la classification des particules polymères,
(vi) la postréticulation en surface des particules polymères classifiées,
(vii) le refroidissement et éventuellement le posttraitement des particules polymères réticulées en surface, le procédé comprenant des étapes de transport pour le produit sous forme de particules formé dans le procédé,
et une machine de transport du groupe des dispositifs de transport continus présentant des moyens de traction étant utilisée lors d'au moins une des étapes de transport,
**caractérisé en ce qu'**on utilise, comme machine de transport, un transporteur à chaîne tubulaire, le transporteur à chaîne tubulaire présentant un tube comme moyen support, une chaîne sur laquelle sont fixés des disques de freinage et des disques d'entraînement en tant que moyens de traction ainsi qu'un poste d'entraînement.

2. Procédé pour la préparation de particules polymères absorbant l'eau selon la revendication 1, **caractérisé en ce que** des machines de transport du groupe des dispositifs de transport continus mécaniques présentant des moyens de traction sont utilisées lors d'au moins une des étapes de transport (a), (b), de préférence lors d'au moins une étape de transport (b), en particulier lors des deux étapes de transport (a), (b),
l'étape de transport (a) se situant avant l'étape (vi) de postréticulation en surface et l'étape de transport (b) se situant après l'étape (vi) de postréticulation en surface.

3. Procédé selon l'une quelconque des revendications 1 ou 2, **caractérisé en ce que** lors de l'étape de transport (c) des machines de transport du groupe des dispositifs de transport continus mécaniques présentant des moyens de traction sont utilisées,
l'étape de transport (c) concernant le transport du produit final superabsorbant fini, c'est-à-dire des particules polymères absorbant l'eau produites, dans des silos à produit fini ou des véhicules à silo.

4. Procédé selon l'une quelconque des revendications 2 ou 3, **caractérisé en ce qu'**au moins l'étape de transport (b), de préférence au moins les étapes de transport (b) et (c), avantageusement les étapes de transport (a), (b) et (c), en particulier l'ensemble des étapes de transport ont lieu avec utilisation de machines de transport du groupe des dispositifs de transport continus mécaniques présentant des moyens de traction.

5. Procédé selon l'une quelconque des revendications 1 à 4, **caractérisé en ce qu'**on utilise en outre au moins dans une des étapes de transport, en plus, un dispositif de transport à vis sans fin.

6. Procédé selon l'une quelconque des revendications 1 à 5, **caractérisé en ce qu'**on ajoute un agent gonflant lors de la polymérisation de la solution ou de la suspension de monomères, de telle sorte qu'un gel polymère insoluble dans l'eau moussé est formé.

7. Procédé selon l'une quelconque des revendications 1 à 6, **caractérisé en ce qu'**on utilise, comme agent gonflant, un gaz, tel qu'en particulier du CO₂, ou un composé présentant un pouvoir de dégagement de gaz, tel qu'en particulier les sels de carbonate.

8. Procédé selon l'une quelconque des revendications 1 à 7, **caractérisé en ce que** la solution ou suspension de monomères contient au moins un tensioactif, de préférence un tensioactif éthyléniquement insaturé.

9. Procédé selon la revendication 8, **caractérisé en ce que** le tensioactif est contenu en une quantité de 0,01 à 5% en poids, de préférence de 0,015 à 2% en poids, plus préférablement de 0,02 à 1% en poids, en particulier de 0,02 à 0,5% en poids dans la solution ou suspension de monomères.

10. Procédé selon l'une quelconque des revendications 1 à 9, **caractérisé en ce que** le monomère a) est l'acide acrylique et **en ce qu'**on utilise, pour la postréticulation en surface dans l'étape vi), un composé qui peut former des liaisons covalentes avec au moins deux groupes acides des particules polymères et **en ce que** les particules polymères sont revêtues après l'étape v), de préférence par des cations métalliques polyvalents, les cations métalliques polyvalents étant en particulier des cations d'aluminium.
